# EUROPÄISCHE PATENTSCHRIFT

(11) **EP 2 303 815 B1**
(45) Veröffentlichungstag und Bekanntmachung des Hinweises auf die Patenterteilung: **26.06.2013**
(21) Anmeldenummer: 09776642.2
(22) Anmeldetag: 22.05.2009
(51) Int. Cl.: C07C 13/62, C09K 19/30, C09K 11/06, H01L 51/00

(54) **ORGANISCHE ELEKTROLUMINESZENZVORRICHTUNG**
ORGANIC ELECTROLUMINESCENT DEVICE
DISPOSITIF ÉLECTROLUMINESCENT ORGANIQUE

(30) Priorität: 29.07.2008 DE 102008035413
(43) Veröffentlichungstag der Anmeldung: 06.04.2011
(73) Patentinhaber: Merck Patent GmbH, 64293 Darmstadt (DE)
(72) Erfinder: KAISER, Joachim, 64293 Darmstadt (DE); VESTWEBER, Horst, 34630 Gilserberg-Winterscheid (DE); LEU, Simone, 67596 Dittelsheim-Hessloch (DE); BUESING, Arne, 65929 Frankfurt am Main (DE); HEIL, Holger, 60389 Frankfurt (DE); STOESSEL, Philipp, 60487 Frankfurt am Main (DE)
(86) Internationale Anmeldenummer: PCT/EP2009/003660
(87) Internationale Veröffentlichungsnummer: WO 2010/012330

(56) Entgegenhaltungen:
- WO-A-2004/041901
- WO-A-2007/140847
- US-A1- 2004 241 491
- SETAYESH S ET AL: "POLYFLUORENES WITH POLYPHENYLENE DENDRON SIDE CHAINS: TOWARD NON-AGGREGATING, LIGHT-EMITTING POLYMERS" JOURNAL OF THE AMERICAN CHEMICAL SOCIETY, AMERICAN CHEMICAL SOCIETY, WASHINGTON, DC. US, Bd. 123, Nr. 5, 1. Januar 2001 (2001-01-01), Seiten 946-953, XP001077861 ISSN: 0002-7863

## Beschreibung

Die vorliegende Erfindung betrifft weiß emittierende organische Elektrolumineszenzvorrichtungen, welche in der blau emittierenden Schicht Dotanden mit bestimmten physikalischen Eigenschaften enthalten.

Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Eine Entwicklung im Bereich organischer Elektrolumineszenzvorrichtungen sind weiß emittierende OLEDs. Diese können entweder für monochrom weiße Displays oder mit Farbfilter für Vollfarbdisplays eingesetzt werden. Weiterhin eignen sie sich für Beleuchtungsanwendungen. Weiß emittierende organische Elektrolumineszenzvorrichtungen auf Basis niedermolekularer Verbindungen weisen im Allgemeinen mindestens zwei Emissionsschichten auf. Häufig weisen sie mindestens drei Emissionsschichten auf, welche blaue, grüne und orange oder rote Emission zeigen. In den Emissionsschichten werden entweder fluoreszierende oder phosphoreszierende Emitter verwendet, wobei die phosphoreszierenden Emitter aufgrund der höheren erreichbaren Effizienz deutliche Vorteile zeigen. Der allgemeine Aufbau einer derartigen weiß emittierenden OLED mit mindestens einer phosphoreszierenden Schicht ist beispielsweise in WO 05/011013 beschrieben. Wegen der höheren erreichbaren Effizienz wäre eine weiß emittierende OLED, welche nur phosphoreszierende Emitterschichten enthält, wünschenswert. Da blau phosphoreszierende Emitter jedoch den gängigen Anforderungen, insbesondere hinsichtlich der Betriebslebensdauer, in der Regel noch nicht entsprechen, werden nach dem Stand der Technik in den meisten Anwendungen Hybrid-OLEDs verwendet, d. h. eine fluoreszierende blaue Emitterschicht kombiniert mit phosphoreszierenden orange oder roten und grünen Emitterschichten (im Fall von Dreifarben-Weiß) oder eine fluoreszierende blaue Emitterschicht kombiniert mit einer phosphoreszierenden gelben bis orange Emitterschicht (im Fall von Zweifarben-Weiß). Dabei ist die blau emittierende Schicht häufig kathodenseitig angeordnet.

Ein grundsätzliches Problem derartiger OLEDs besteht darin, dass die kathodenseitig angeordnete blaue Emissionsschicht eine besonders elektronenreiche Umgebung sieht. Als blaue Dotanden werden gemäß dem Stand der Technik im Allgemeinen Arylamine mit kondensierten Aromaten, beispielsweise Chrysenamine oder Pyrenamine, verwendet, die häufig ein Stabilitätsproblem gegenüber Elektronen aufweisen, was zu einer Verringerung der Lebensdauer führt. Daher ist die Lebensdauer des blauen Emitters limitierend für die Lebensdauer der weiß emittierenden Elektrolumineszenzvorrichtung. Hier besteht daher Verbesserungsbedarf. Insbesondere sollten blau emittierende Dotanden gefunden werden, die eine hohe Stabilität gegenüber einer elektronenreichen Umgebung aufweisen und somit zu einer verbesserten Lebensdauer der weiß emittierenden Vorrichtung führen.

In WO 2007/140847 ist eine OLED offenbart, welche ein Dibenzofluoren-Derivat enthält. In dem Dokument ist jedoch nicht offenbart, dass die OLED eine blau emittierende Schicht enthält, die kathodenseitig von einer weiteren emittierenden Schicht angeordnet ist. Weiterhin ist in dem Dokument nicht offenbart, dass bestimmte HOMO-Werte für den blau emittierenden Dotanden der blau emittierenden Schicht einzuhalten sind.

In US 2004/0241491 ist eine OLED offenbart, welche in dieser Reihenfolge eine Anode, eine erste Emitterschicht, eine zweite, blau emittierende Schicht und eine Kathode enthält. Als Dotanden der blau emittierenden Schicht sind insbesondere Stylrylamine offenbart. In dem Dokument ist jedoch nicht offenbart, dass die Emitterschicht einen blau emittierenden Dotanden und einen Host enthält, wobei der blau emittierende Dotand in einem Anteil von 0.1 bis 10 Vol.-% vorliegt und ein HOMO kleiner als -5.2 eV aufweist.

Überraschend wurde gefunden, dass eine weiß emittierende organische Elektrolumineszenzvorrichtung, welche die blau emittierende Schicht kathodenseitig angeordnet enthält, eine deutlich verbesserte Lebensdauer aufweist, wenn als blau emittierender Dotand eine Verbindung verwendet wird, deren HOMO (highest occupied molecular orbital) kleiner als -5.2 eV ist.

Gegenstand der Erfindung ist somit eine organische Elektrolumineszenzvorrichtung, enthaltend in dieser Reihenfolge eine Anode, eine erste Emitterschicht, eine zweite Emitterschicht, welches eine blau emittierende Schicht ist, wobei die blau emittierende Schicht ein Hostmaterial in einem Anteil von 90 - 99.9 Vol.-% und einen Dotanden in einem Anteil von 0.1 - 10 Vol.-% enthält, und eine Kathode, dadurch gekennzeichnet, dass der Dotand ein HOMO von kleiner als -5.2 eV aufweist.

Dabei wird das HOMO bestimmt, wie hinten in Beispiel 1 allgemein beschrieben.

In einer bevorzugten Ausführungsform der Erfindung handelt es sich um eine weiß emittierende organische Elektrolumineszenzvorrichtung. Diese ist dadurch charakterisiert, dass sie Licht mit CIE-Farbkoordinaten im Bereich von 0.28/0.29 bis 0.45/0.41 emittiert.

Die erfindungsgemäße organische Elektrolumineszenzvorrichtung enthält, wie oben beschrieben, Anode, Kathode und mindestens zwei emittierende Schichten, welche zwischen der Anode und der Kathode angeordnet sind. Die organische Elektrolumineszenzvorrichtung muss nicht notwendigerweise nur Schichten enthalten, welche aus organischen oder metallorganischen Materialien aufgebaut sind. So ist es auch möglich, dass Anode, Kathode und/oder eine oder mehrere Schichten anorganische Materialien enthalten oder ganz aus anorganischen Materialien aufgebaut sind.

Wenn die organische Elektrolumineszenzvorrichtung genau zwei emittierende Schichten aufweist, ist die erste Emitterschicht, also die Emitterschicht auf Anodenseite, bevorzugt eine gelb oder orange emittierende Emitterschicht, wobei es sich bevorzugt um eine phosphoreszierende Emitterschicht handelt.

In einer bevorzugten Ausführungsform der Erfindung weist die erfindungsgemäße Elektrolumineszenzvorrichtung mindestens drei emittierende Schichten auf.

Wenn die organische Elektrolumineszenzvorrichtung drei emittierende Schichten aufweist, so ist eine dieser Schichten bevorzugt eine rot oder orange emittierende Emitterschicht und eine der Schichten eine grün emittierende Emitterschicht. In einer bevorzugten Ausführungsform der Erfindung liegt die rot oder orange emittierende Schicht auf Anodenseite und die grün emittierende Schicht liegt zwischen der rot emittierenden Schicht und der blau emittierenden Schicht. In einer bevorzugten Ausführungsform der Erfindung handelt es sich bei der rot oder orange emittierenden Schicht und/oder bei der grün emittierenden Schicht um phosphoreszierende Schichten. Besonders bevorzugt handelt es sich sowohl bei der rot oder orange emittierenden Schicht wie auch bei der grün emittierenden Schicht um phosphoreszierende Schichten.

Es ist auch möglich, dass die organische Elektrolumineszenzvorrichtung mehr als drei Emitterschichten aufweist.

In einer bevorzugten Ausführungsform der Erfindung ist zwischen der blau emittierenden Schicht und der Kathode keine weitere emittierende Schicht enthalten.

In einer bevorzugten Ausführungsform der Erfindung handelt es sich bei der blau emittierenden Schicht um eine fluoreszierende Schicht, d. h. bei dem Dotanden handelt es sich um einen fluoreszierenden Dotanden.

Dabei wird unter einer gelb emittierenden Schicht eine Schicht verstanden, deren Photolumineszenzmaximum im Bereich von 540 bis 570 nm liegt. Unter einer orange emittierenden Schicht wird eine Schicht verstanden, deren Photolumineszenzmaximum im Bereich von 570 bis 600 nm liegt. Unter einer rot emittierenden Schicht wird eine Schicht verstanden, deren Photolumineszenzmaximum im Bereich von 600 bis 750 nm liegt. Unter einer grün emittierenden Schicht wird eine Schicht verstanden, deren Photolumineszenzmaximum im Bereich von 490 bis 540 nm liegt. Unter einer blau emittierenden Schicht wird eine Schicht verstanden, deren Photolumineszenzmaximum im Bereich von 440 bis 490 nm liegt. Dabei wird das Photolumineszenzmaximum durch Messung des Photolumineszenzspektrums der Schicht mit einer Schichtdicke von 50 nm bestimmt.

Besonders bevorzugt weist die erfindungsgemäße organische Elektrolumineszenzvorrichtung den folgenden Aufbau auf: Anode / orange oder rot phosphoreszierende Emitterschicht / grün phosphoreszierende Emitterschicht / blau fluoreszierende Emitterschicht / Kathode. Die Elektrolumineszenzvorrichtung kann auch weitere Schichten aufweisen, die oben nicht aufgeführt sind.

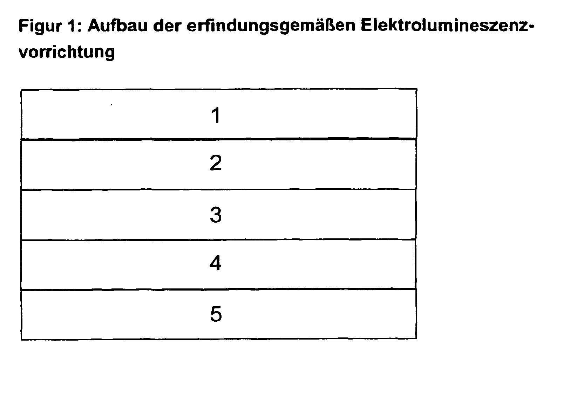

Dieser allgemeine Device-Aufbau ist schematisch in Figur 1 dargestellt. Dabei steht die Schicht 1 für die Anode, die Schicht 2 für die rot phosphoreszierende Emitterschicht, die Schicht 3 für die grün phosphoreszierende Emitterschicht, die Schicht 4 für die blau fluoreszierende Emitterschicht und die Schicht 5 für die Kathode. Die Elektrolumineszenzvorrichtung kann auch weitere Schichten aufweisen, die nicht in Figur 1 abgebildet sind.

Eine phosphoreszierende Verbindung im Sinne dieser Erfindung, wie sie in den phosphoreszierenden Emitterschichten der erfindungsgemäßen organischen Vorrichtung vorliegt, ist eine Verbindung, welche bei Raumtemperatur Lumineszenz aus einem angeregten Zustand mit höherer Spinmultiplizität zeigt, also einem Spinzustand > 1, insbesondere aus einem angeregten Triplettzustand. Im Sinne dieser Erfindung sollen alle lumineszierenden Übergangsmetallkomplexe der zweiten und dritten Übergangsmetallreihe, insbesondere alle lumineszierenden Iridium- und Platinverbindungen, als phosphoreszierende Verbindungen angesehen werden.

Eine fluoreszierende Verbindung im Sinne dieser Erfindung, wie sie in der blau fluoreszierenden Emitterschicht vorliegt, ist eine Verbindung, welche bei Raumtemperatur Lumineszenz aus einem angeregten Singulett-Zustand zeigt. Im Sinne dieser Erfindung sollen insbesondere alle lumineszierenden Verbindungen, die nur aus den Elementen C, H, N, O, S, F, B und P aufgebaut sind, als fluoreszierende Verbindungen verstanden werden.

Im Folgenden wird der blau emittierende Dotand, der in der blau emittierenden Emitterschicht vorliegt, genauer beschrieben:

Wie oben beschrieben, weist der blaue Dotand ein HOMO (highest occupied molecular orbital) von kleiner als -5.2 eV auf. Bevorzugt ist das HOMO kleiner als -5.3 eV, besonders bevorzugt kleiner als -5.4 eV.

Weiterhin bevorzugt weist der blaue Dotand ein LUMO (lowest unoccupied molecular orbital) von kleiner als -2.3 eV auf, besonders bevorzugt von kleiner als -2.5 eV. Dabei wird das LUMO bestimmt, wie hinten in Beispiel 1 allgemein beschrieben.

Wie oben beschrieben, liegt der blaue Dotand in einer Konzentration von 0.1 - 10 Vol.% in der blau emittierenden Schicht vor. Bevorzugt ist ein Anteil von 0.2 - 7 Vol.-%, besonders bevorzugt ein Anteil von 0.5 - 5 Vol.-%, ganz besonders bevorzugt ein Anteil von 0.8 - 3 Vol.-%.

In einer bevorzugten Ausführungsform der Erfindung enthält der blaue Dotand keine Diarylaminogruppen, besonders bevorzugt überhaupt keine Aminogruppen. Diese Bevorzugung ist mit der vergleichsweise geringen Stabilität der Diarylaminogruppen gegenüber Elektronen begründet.

In einer bevorzugten Ausführungsform der Erfindung ist der blaue Dotand eine Verbindung gemäß der folgenden Formel (1), 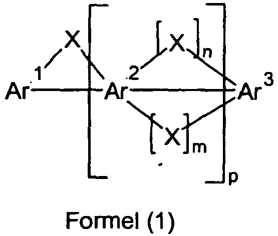 wobei für die verwendeten Symbole und Indizes gilt:
- Ar¹, Ar², Ar³: ist bei jedem Auftreten gleich oder verschieden eine Aryl - oder Heteroarylgruppe mit 5 bis 30 aromatischen Ringatomen, die mit einem oder mehreren Resten R¹ substituiert sein kann;
- X: ist bei jedem Auftreten gleich oder verschieden eine Gruppe, ausgewählt aus BR², C(R²)₂, Si(R²)₂, C=O, C=NR², C=C(R²)₂, O, S, S=O, SO₂, NR², PR², P(=O)R² oder P(=S)R²;
- R¹, R²: ist bei jedem Auftreten gleich oder verschieden H, D, F, Cl, Br, I, C(=O)Ar⁴, P(=O)(Ar⁴)₂, S(=O)Ar⁴, S(=O)₂Ar⁴, CR²=CR²Ar⁴, CHO, CR³=C(R³)₂, CN, NO₂, Si(R³)₃, B(OR³)₂, B(R³)₂, B(N(R³)₂)₂, OSO₂R³, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R³ substituiert sein kann, wobei jeweils eine oder mehrere nicht benachbarte CH₂-Gruppen durch R³C=CR³, C≡C, Si(R³)₂, Ge(R³)₂, Sn(R³)₂, C=O, C=S, C=Se, C=NR ³ P(=O)R³, SO, SO₂, NR³, O, S oder CONR³ ersetzt sein können und wobei ein oder mehrere H-Atome durch F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R³ substituiert sein kann, oder eine Kombination dieser Systeme; dabei können zwei oder mehrere Substituenten R¹ bzw. R² auch miteinander ein mono - oder polycyclisches, aliphatisches oder aromatisches Ringsystem bilden;
- R³: ist bei jedem Auftreten gleich oder verschieden H, D oder ein aliphatischer oder aromatischer Kohlenwasserstoffrest mit 1 bis 20 C-Atomen;
- Ar⁴: ist bei jedem Auftreten gleich oder verschieden ein aromatisches oder heteroaromatisches Ringsystem mit 5-30 aromatischen Ringatomen, das mit einem oder mehreren nicht-aromatischen Resten R¹ substituiert sein kann; dabei können auch zwei Reste Ar am selben Stickstoff- oder Phosphoratom durch eine Einfachbindung oder eine Brücke X miteinander verknüpft sein;
- m, n: sind 0 oder 1, mit der Maßgabe, dass m + n = 1 ist;
- p: ist 1, 2, 3, 4, 5 oder 6;
dabei bilden Ar¹, Ar² und X zusammen einen Fünfring oder einen Sechsring und Ar², Ar³ und X bilden zusammen einen Fünfring oder einen Sechsring.

Dabei beträgt die Summe aller π-Elektronen der Gruppen Ar¹, Ar² und Ar³ bevorzugt mindestens 28, wenn p = 1 ist, und mindestens 34, wenn p = 2 ist, und mindestens 40, wenn p = 3 ist, und mindestens 46, wenn p = 4 ist und mindestens 52, wenn p = 5 ist und mindestens 58, wenn p = 6 ist.

Die Summe aller π-Elektronen der Gruppen Ar¹, Ar² und Ar³ zu ermitteln, ist für den Fachmann offensichtlich. So steht in einer Arylgruppe jede Doppelbindung (wobei es sich um delokalisierte Doppelbindungen handelt) für zwei π-Elektronen, so dass beispielsweise Benzol 6 π-Elektronen, Naphthalin 10 π-Elektronen, Anthracen und Phenanthren 14 π-Elektronen, Pyren 16 π-Elektronen, Naphthacen, Benzanthracen und Chrysen 18 π-Elektronen und Perylen 20 π-Elektronen aufweisen. In einer Arylgruppe stimmt die Zahl der π-Elektronen mit der Zahl der C-Atome im aromatischen Ringsystem überein. Auch in Heteroaromaten trägt jede Doppelbindung (auch hier handelt es sich um delokalisierte Doppelbindungen) zwei π-Elektronen bei, wobei diese delokalisierten Doppelbindungen entweder zwischen zwei Kohlenstoffatomen, zwischen Kohlenstoff und Stickstoff oder zwischen zwei Stickstoffatomen gebildet werden können. Weiterhin trägt in Fünfring-Heteroarylgruppen jeweils das Heteroatom, das formal nicht in einer Doppelbindung gebunden ist (also beispielsweise der Stickstoff im Pyrrol, der Sauerstoff im Furan oder der Schwefel im Thiophen) ebenfalls über das freie Elektronenpaar zwei π-Elektronen zum gesamten π-Elektronensystem bei. Daher weisen beispielsweise Pyridin, Pyrazin, Pyrimidin und Pyridazin jeweils 6 π-Elektronen, Chinolin und Isochinolin 10 π-Elektronen, Phenanthrolin 14 π-Elektronen, Pyrrol, Imidazol, Pyrazol, Thiophen, Thiazol und Furan jeweils 6 π-Elektronen, Indol, Benzimidazol, Benzothiophen und Benzofuran jeweils 10 π-Elektronen und Carbazol, Dibenzothiophen und Dibenzofuran jeweils 14 π-Elektronen auf.

Unter einer Arylgruppe bzw. einer Heteroarylgruppe im Sinne dieser Erfindung wird eine aromatische Gruppe bzw. heteroaromatische Gruppe mit einem gemeinsamen aromatischen Elektronensystem verstanden, wobei eine Arylgruppe 6 bis 30 C-Atome und eine Heteroarylgruppe 2 bis 30 C-Atome und insgesamt mindestens 5 aromatische Ringatome umfasst. Die Heteroatome sind bevorzugt ausgewählt aus N, O und/oder S. Dies kann im Sinne dieser Erfindung ein einfacher Homo- oder Heterocyclus sein, beispielsweise Benzol, Pyridin, Thiophen, etc., oder es kann eine kondensierte Aryl- oder Heteroarylgruppe sein, in der mindestens zwei aromatische oder heteroaromatische Ringe, beispielsweise Benzolringe, miteinander "verschmolzen", d. h. durch Anellierung einander ankondensiert sind, also mindestens eine gemeinsame Kante und dadurch auch ein gemeinsames aromatisches System aufweisen. Diese Aryl- oder Heteroarylgruppe kann substituiert oder unsubstituiert sein; ebenso können gegebenenfalls vorhandene Substituenten weitere Ringsysteme bilden. So sind beispielsweise Systeme wie Naphthalin, Anthracen, Phenanthren, Pyren, etc. als Arylgruppen und Chinolin, Acridin, Benzothiophen, Carbazol, etc. als Heteroarylgruppen im Sinne dieser Erfindung zu sehen, während beispielsweise Biphenyl, Fluoren, Spirobifluoren, etc. keine Arylgruppen darstellen, da es sich hierbei um separate aromatische Elektronensysteme handelt.

Ein aromatisches Ringsystem im Sinne dieser Erfindung enthält 6 bis 60 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe der C-Atome und der Heteroatome mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine kurze, nicht-aromatische Einheit (weniger als 10 % der von H verschiedenen Atome, bevorzugt weniger als 5 % der von H verschiedenen Atome), wie z. B. ein C-, N- oder O-Atom, unterbrochen sein können. So sollen beispielsweise auch Systeme wie 9,9'-Spirobifluoren, 9,9-Diaryl-fluoren, Triarylamin, Diarylether, etc. als aromatische Ringsysteme im Sinne dieser Erfindung verstanden werden.

Im Rahmen der vorliegenden Erfindung werden unter einer C₁- bis C₄₀-Alkylgruppe, in der auch einzelne H-Atome oder CH₂-Gruppen durch die oben genannten Gruppen substituiert sein können, besonders bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, tert-Pentyl, 2-Pentyl, Cyclopentyl, n-Hexyl, s-Hexyl, tert-Hexyl, 2-Hexyl, 3-Hexyl, Cyclohexyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer C₁- bis C₄₀-Alkoxygruppe werden besonders bevorzugt Methoxy, Ethoxy, n-Propoxy, i-Propoxy, n-Butoxy, i-Butoxy, s-Butoxy, t-Butoxy oder 2-Methylbutoxy verstanden. Unter einer C₂-C₂₄ Aryl- oder Heteroarylgruppe, die je nach Verwendung monovalent oder bivalent sein kann, die noch jeweils mit den oben genannten Resten R¹ substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Benzfluoranthen, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol. Unter aromatischen und heteroaromatischen Ringsystemen im Sinne dieser Erfindung werden außer den oben genannten Aryl- und Heteroarylgruppen insbesondere Biphenylen, Terphenylen, Fluoren, Benzofluoren, Dibenzofluoren, Spirobifluoren, Dihydrophenanthren, Tetrahydropyren, cis- oder trans-Indenofluoren, cis- oder trans-Monobenzoindenofluoren oder cis- oder trans-Dibenzoindenofluoren verstanden.

In einer bevorzugten Ausführungsform der Erfindung ist der Index p = 1, 2 oder 3, besonders bevorzugt 1 oder 2, ganz besonders bevorzugt 1.

In einer bevorzugten Ausführungsform der Erfindung beträgt die Summe aller π-Elektronen der Gruppen Ar¹, Ar² und Ar³ zwischen 28 und 50, besonders bevorzugt zwischen 28 und 46, ganz besonders bevorzugt zwischen 28 und 42, insbesondere zwischen 28 und 36, wenn p = 1 ist, und beträgt zwischen 34 und 56, besonders bevorzugt zwischen 34 und 52, ganz besonders bevorzugt zwischen 34 und 48, insbesondere zwischen 34 und 40, wenn p = 2 ist, und beträgt zwischen 40 und 62, besonders bevorzugt zwischen 40 und 58, ganz besonders bevorzugt zwischen 40 und 54, insbesondere zwischen 40 und 46, wenn p = 3 ist.

Bevorzugt sind weiterhin Verbindungen gemäß Formel (1), in denen die Symbole Ar¹, Ar² und Ar³ gleich oder verschieden bei jedem Auftreten für eine Aryl- oder Heteroarylgruppe mit 5 bis 22 aromatischen Ringatomen, insbesondere mit 5 bis 18 aromatischen Ringatomen, stehen. Dabei sind die Gruppen Ar¹, Ar² und Ar³ unabhängig voneinander besonders bevorzugt ausgewählt aus der Gruppe bestehend aus Benzol, Naphthalin, Anthracen, Phenanthren, Fluoranthen, Naphthacen, Benzanthracen, Chrysen, Pyren, Benzfluoranthen, Triphenylen, Perylen, Dibenzanthracen, Benzpyren, Picen, Pentacen, Pentaphen, Benzphenanthren, Pyridin, Pyrazin, Pyrimidin, Pyridazin, Chinolin, Isochinolin, Phenanthrolin, Acridin. Besonders bevorzugt stehen die Symbole Ar¹, Ar² und Ar³ bei jedem Auftreten gleich oder verschieden für eine Arylgruppe mit 6 bis 18 aromatischen Ringatomen, insbesondere ausgewählt aus Benzol, Naphthalin, Anthracen, Phenanthren, Fluoranthen, Naphthacen, Benzanthracen, Chrysen, Pyren, Benzfluoranthen und Triphenylen.

Besonders bevorzugte Gruppen Ar¹ und Ar³, welche mit Ar² einen Fünfring bilden, sind die im Folgenden aufgeführten Gruppen der Formeln (2) bis (85), welche jeweils durch einen oder mehrere Reste R¹ substituiert sein können. Dabei steht das Symbol * für die Position der Verknüpfung von Ar¹ bzw. Ar³ mit Ar² und das Symbol # steht für die Position der Verknüpfung von Ar¹ bzw. Ar³ mit X.

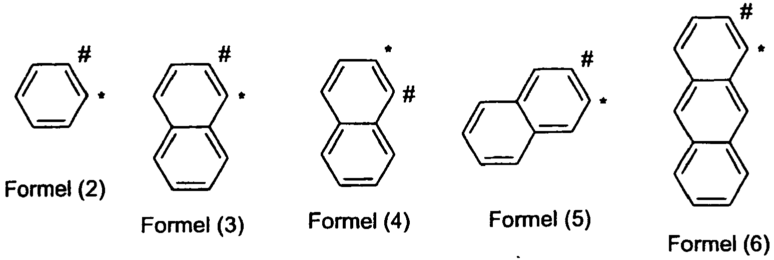

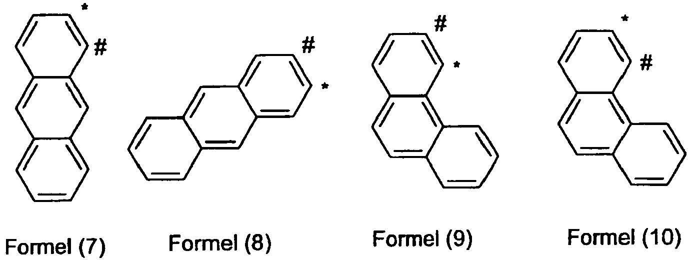

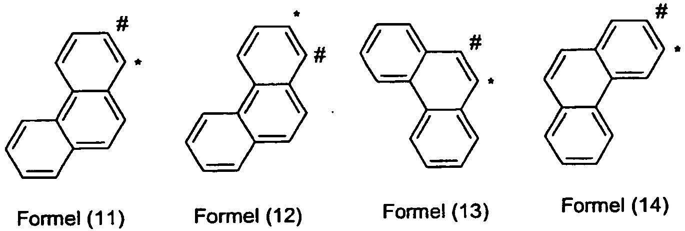

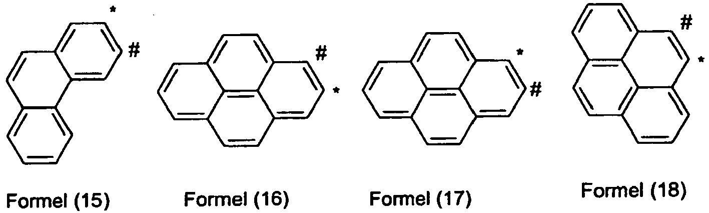

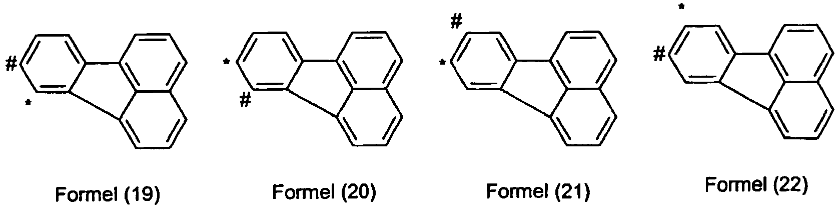

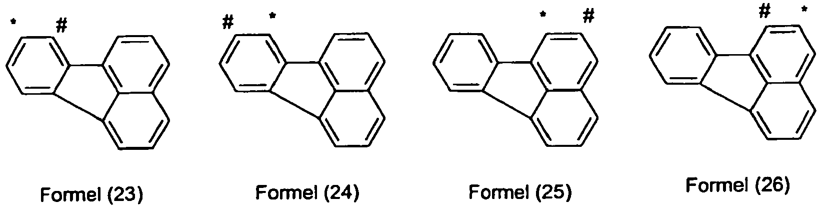

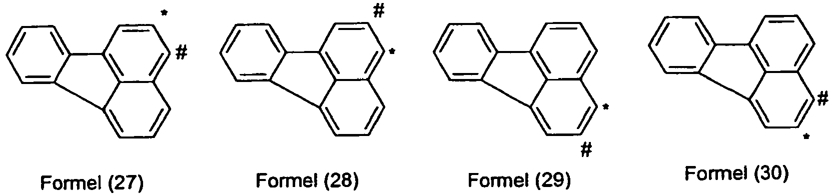

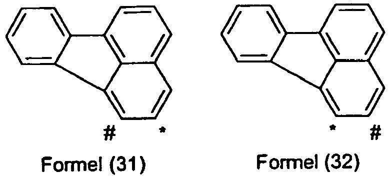

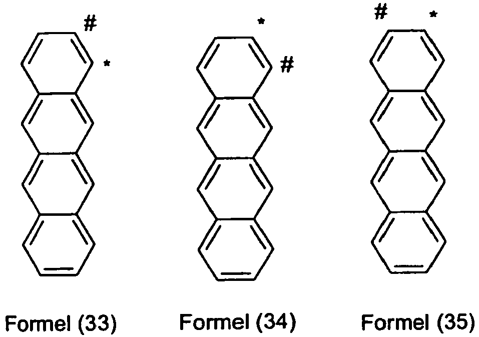

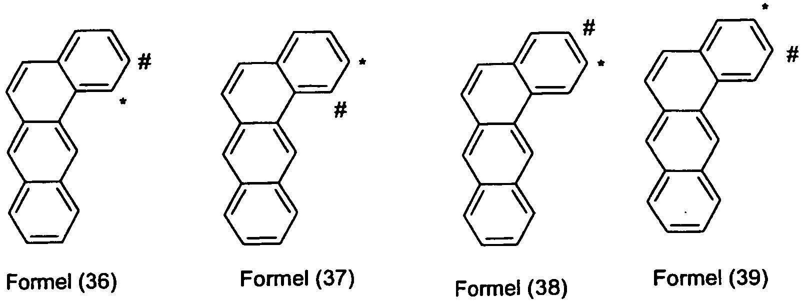

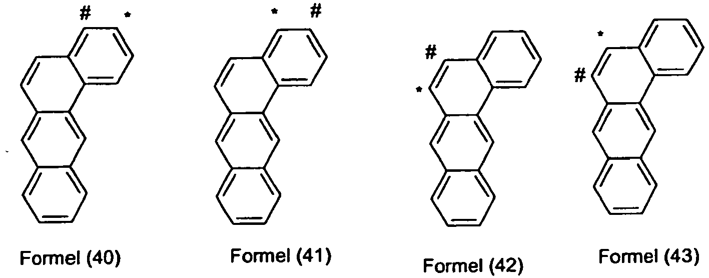

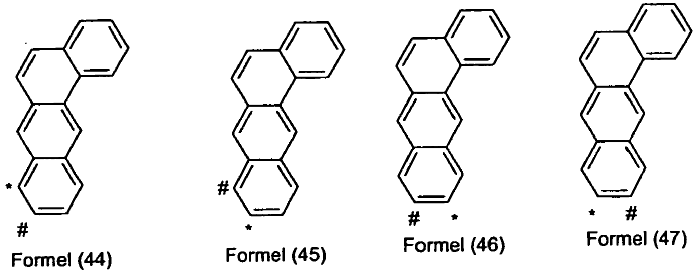

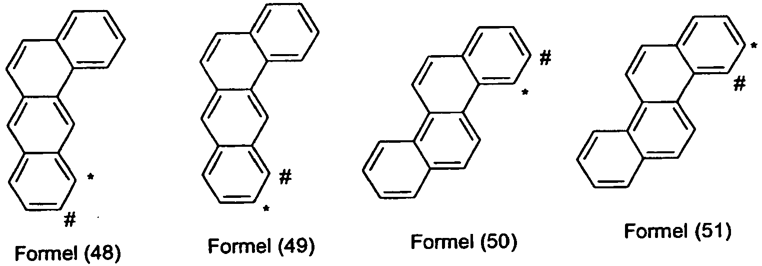

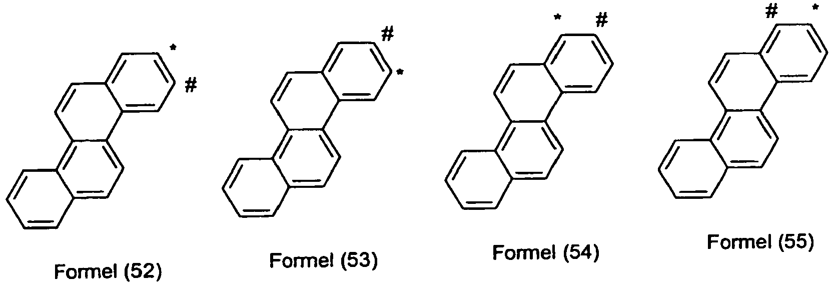

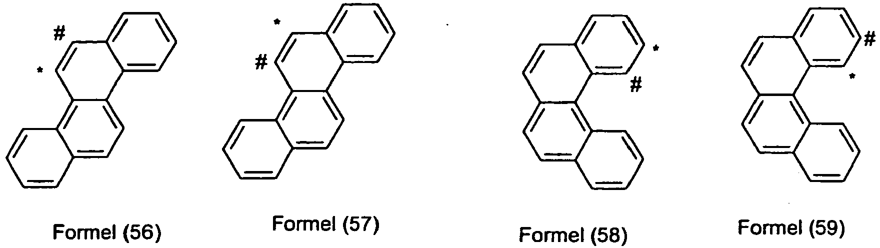

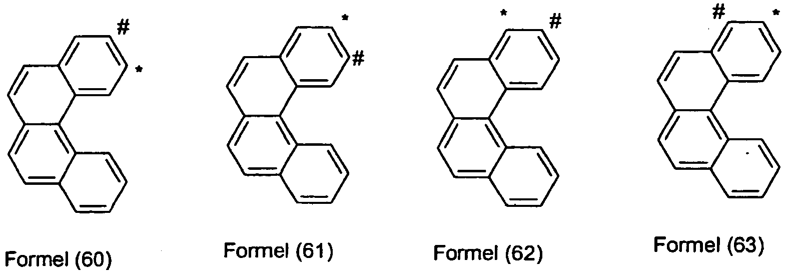

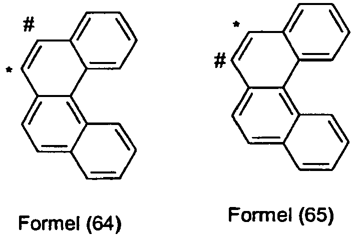

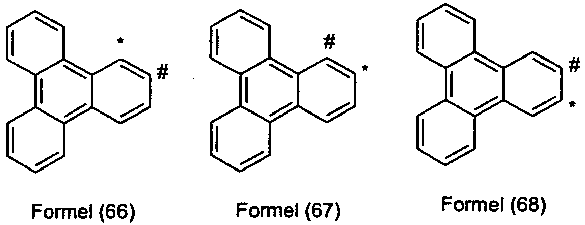

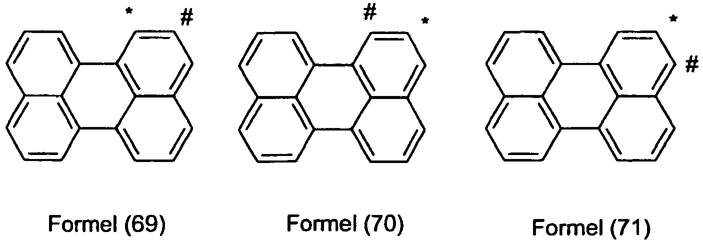

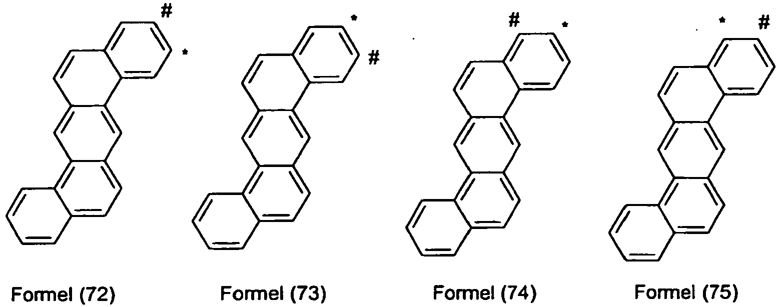

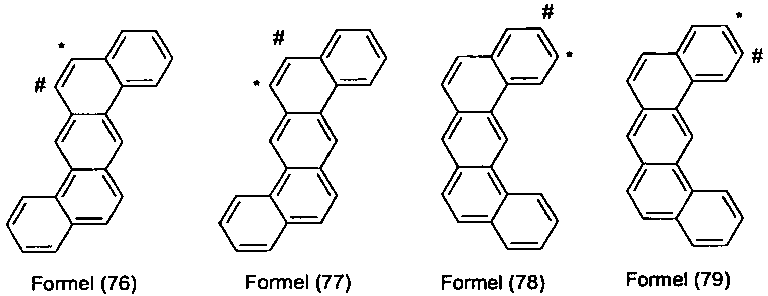

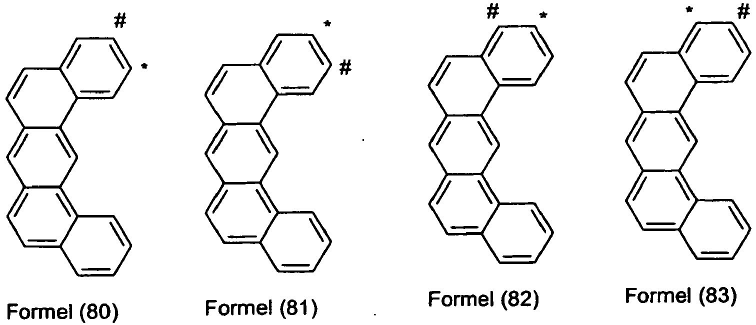

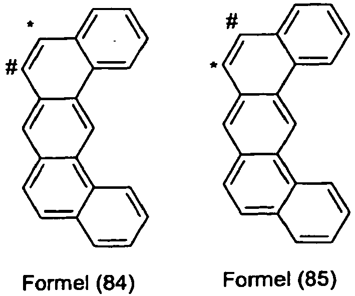

Besonders bevorzugte Gruppen Ar² sind die im Folgenden aufgeführten Gruppen der Formeln (86) bis (110), welche jeweils durch einen oder mehrere Reste R¹ substituiert sein können. Dabei steht das Symbol * für die Position der Verknüpfung von Ar² mit Ar¹ bzw. Ar³ und das Symbol # steht für die Position der Verknüpfung von Ar² mit X.

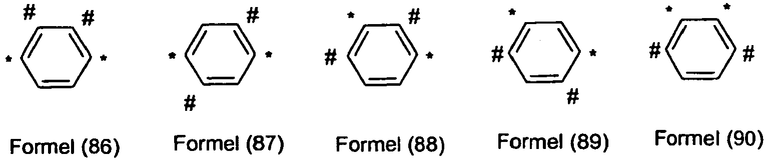

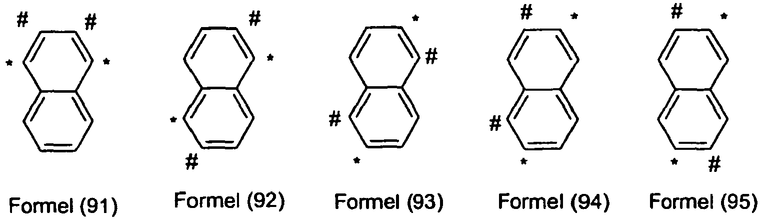

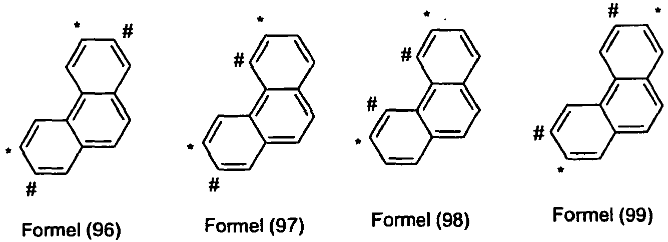

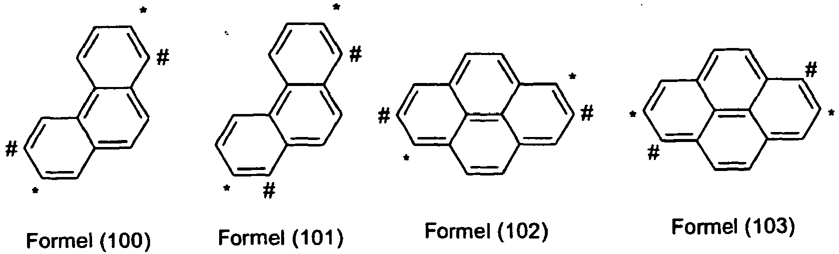

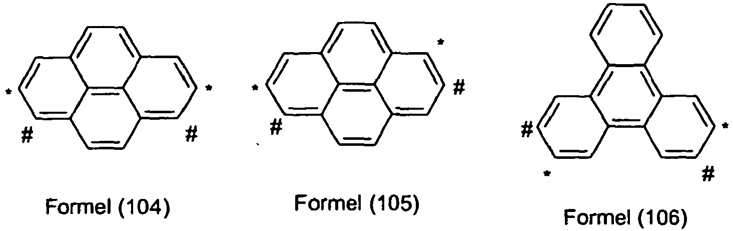

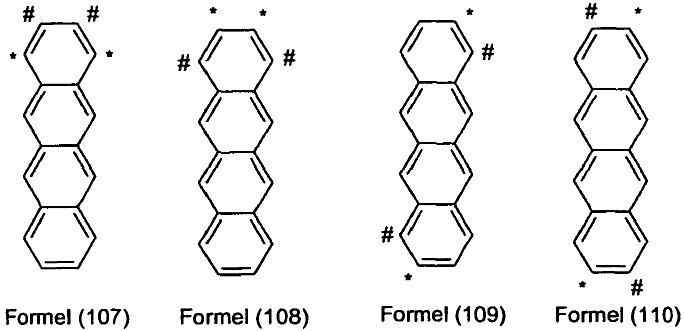

Analog ist die Bildung von Sechsringen aus zwei der oben abgebildeten Formeln und der Gruppe X möglich.

Bevorzugt sind weiterhin Verbindungen, in denen mindestens eine der Gruppen Ar¹, Ar² bzw. Ar³ mindestens 3 kondensierte Ringe, also mindestens 14 π-Elektronen aufweist, wobei diese Gruppen bevorzugt aus den oben abgebildeten Formeln ausgewählt sind. Besonders bevorzugt weist mindestens eine der Gruppen Ar¹, Ar² bzw. Ar³ mindestens 4 kondensierte Ringe, also mindestens 16 π-Elektronen auf. Ganz besonders bevorzugt weist mindestens eine der Gruppen Ar¹, Ar² bzw. Ar³ mindestens 4 kondensierte Ringe auf, also mindestens 16 π-Elektronen, und mindestens eine der anderen beiden Gruppen Ar¹, Ar² bzw. Ar³ weist mindestens 2 kondensierte Ringe, also mindestens 10 π-Elektronen, auf.

Bevorzugt sind weiterhin Verbindungen gemäß Formel (1), in denen das Symbol X gleich oder verschieden bei jedem Auftreten ausgewählt ist aus der Gruppe bestehend aus B(R²), C(R²)₂, Si(R²)₂, O, S oder N(R²), besonders bevorzugt C(R²)₂, S oder N(R²). Ganz besonders bevorzugt stehen alle Symbole X gleich oder verschieden bei jedem Auftreten für C(R²)₂. Dabei steht R² bevorzugt für eine Alkyl- oder Arylgruppe, wie oben definiert.

Besonders bevorzugt sind Ar¹, Ar² und Ar³ aus den oben genannten Formeln gewählt und X steht gleichzeitig gleich oder verschieden bei jedem Auftreten für C(R²)₂. Dabei steht R² bevorzugt für eine Alkyl- oder Arylgruppe.

Besonders bevorzugt sind Verbindungen gemäß Formel (1), ausgewählt aus den Formeln (111) bis (141), wobei die aromatischen Systeme jeweils auch durch einen oder mehrere Reste R¹ substituiert sein können:

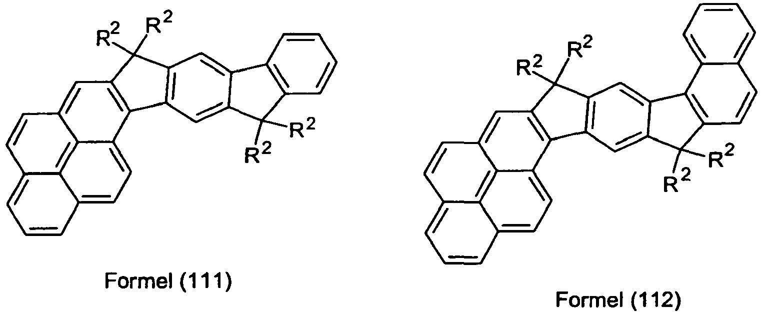

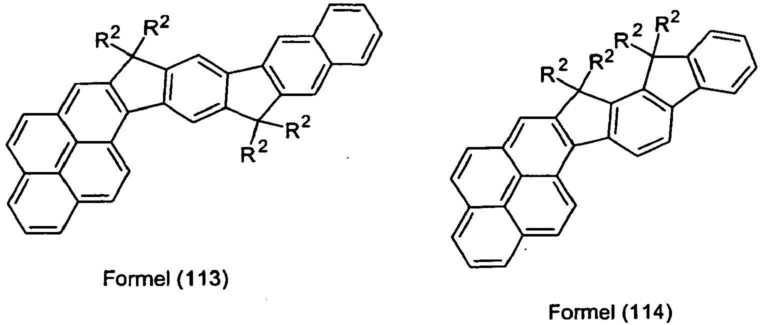

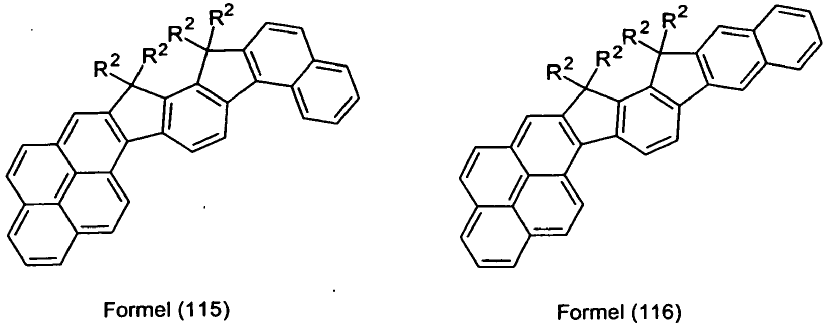

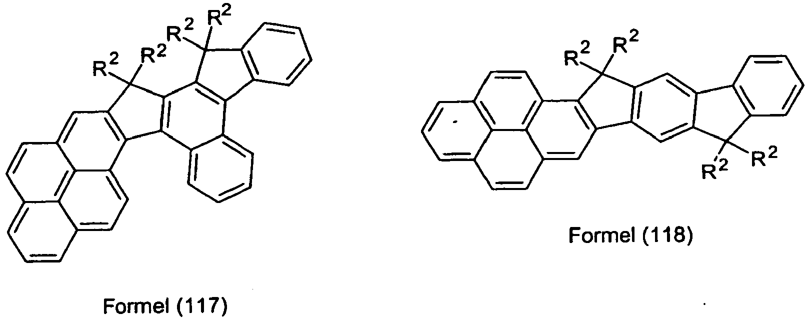

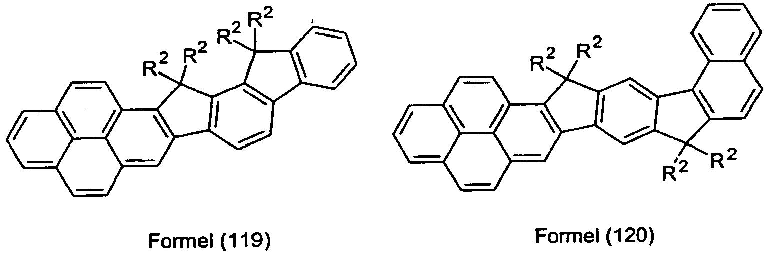

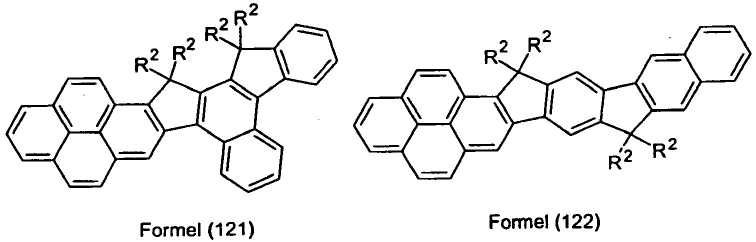

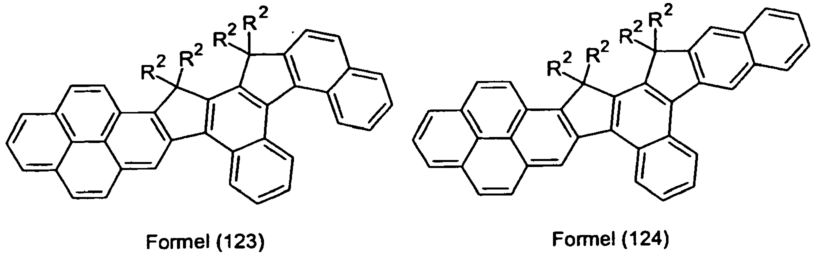

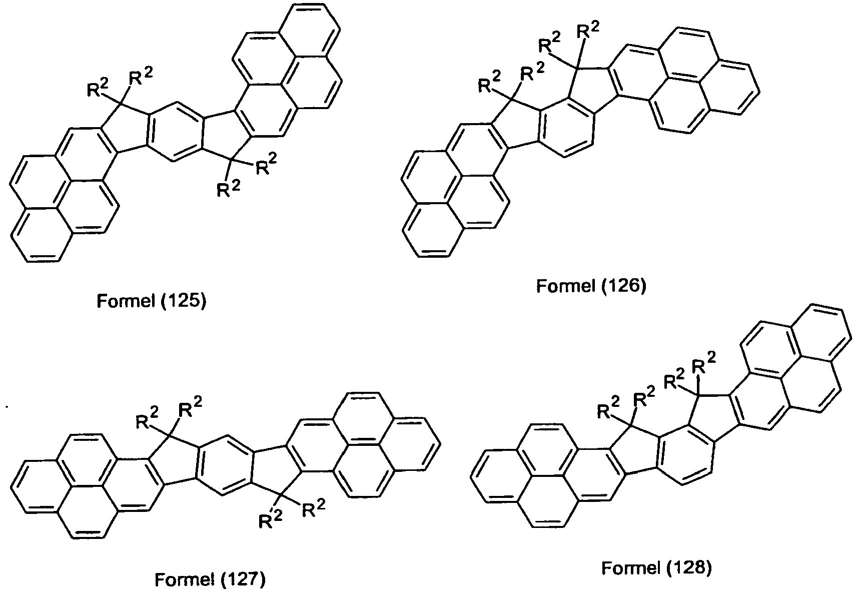

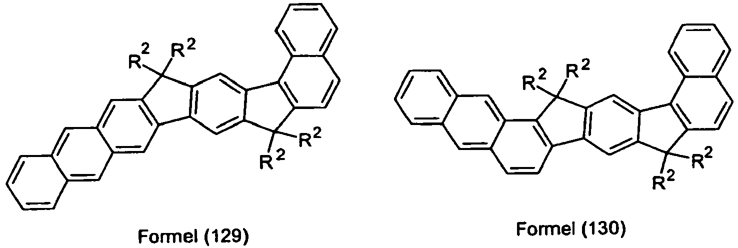

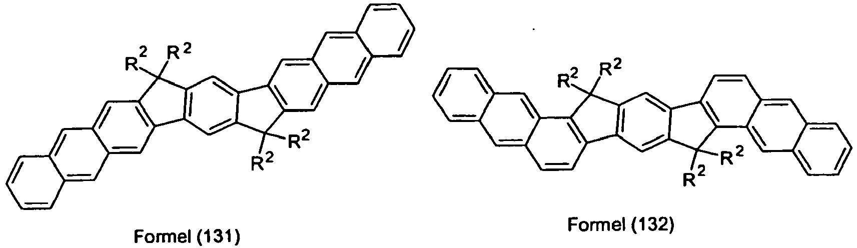

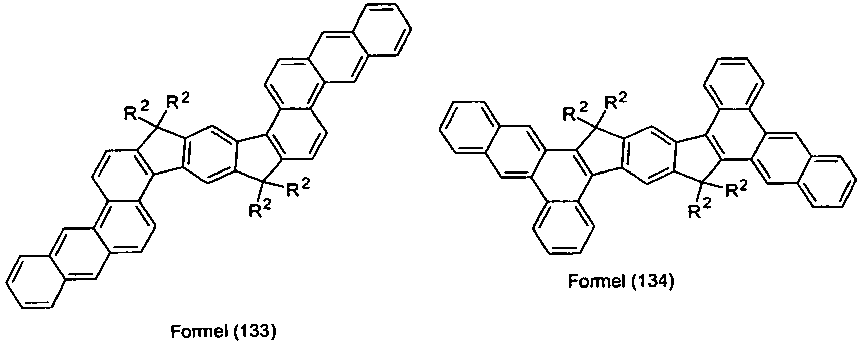

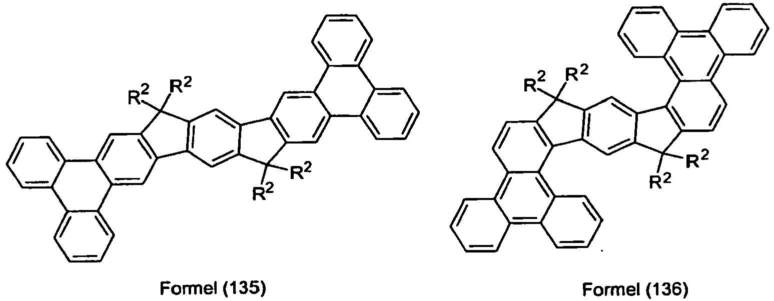

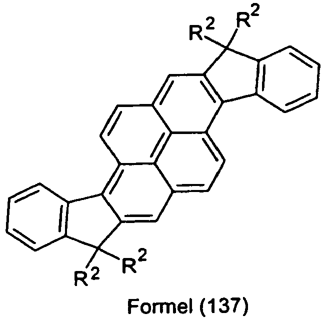

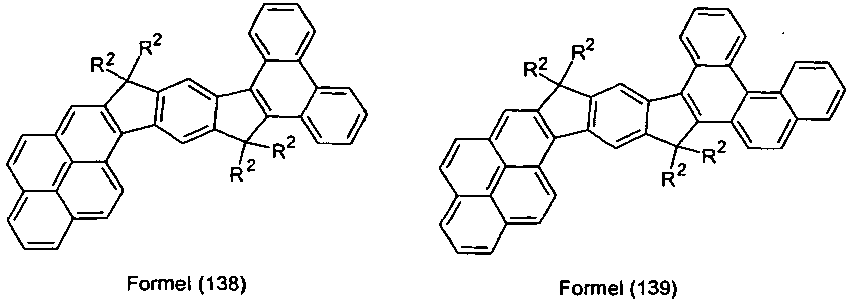

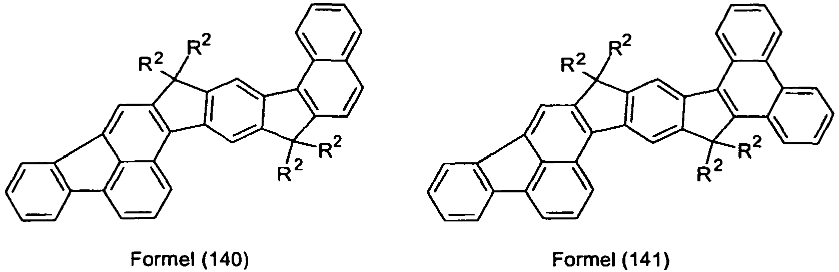

Bevorzugt sind weiterhin Verbindungen gemäß Formel (1), in denen das Symbol R¹, welches als Substituent an Ar¹, Ar² oder Ar³ gebunden sein kann, bei jedem Auftreten gleich oder verschieden ausgewählt ist aus der Gruppe bestehend aus H, D, F, Si(R³)₃, geradkettigen Alkyl- oder Alkoxygruppen mit 1 bis 10 C-Atomen oder verzweigten oder cyclischen Alkyl - oder Alkoxygruppen mit 3 bis 10 C-Atomen, die jeweils mit einem oder mehreren Resten R³ substituiert sein kann, wobei jeweils eine oder mehrere nicht benachbarte CH₂-Gruppen durch R³C=CR³ oder O ersetzt sein können und wobei ein oder mehrere H-Atome durch F ersetzt sein können, oder aromatischen oder heteroaromatischen Ringsystemen mit 5 bis 40 aromatischen Ringatomen oder einer Kombination dieser Systeme; dabei können zwei oder mehrere Substituenten R¹ auch miteinander ein mono- oder polycyclisches, aliphatisches oder aromatisches Ringsystem bilden. Besonders bevorzugt ist der Substituent R¹ ausgewählt aus H, D, geradkettigen Alkylgruppen mit 1 bis 6 C-Atomen, verzweigten oder cyclischen Alkylgruppen mit 3 bis 6 C-Atomen oder einem aromatischen oder heteroaromatischen Ringsystem mit 5 bis 24 aromatischen Ringatomen; dabei können zwei oder mehrere Substituenten R¹ auch miteinander ein mono- oder polycyclisches Ringsystem bilden. Ganz besonders bevorzugt ist der Substituent R¹ ausgewählt aus H, D, Alkylgruppen, ausgewählt aus der Gruppe bestehend aus Methyl, Ethyl, n-Propyl, iso-Propyl, n-Butyl, iso-Butyl, tert-Butyl, Cyclopentyl oder Cyclohexyl, insbesondere Methyl oder tert-Butyl, und aromatischen oder heteroaromatischen Ringsystemen, ausgewählt aus der Gruppe bestehend aus unsubstituiertem oder durch R³ substituiertem Phenyl oder Naphthyl, Benzimidazol, welches auch durch Phenyl oder andere Reste R³ substituiert sein kann, Phenyl-Benzimidazol, wobei das Benzimidazol auch durch Phenyl oder andere Reste R³ substituiert sein kann, oder Triazin, welches auch durch Phenyl oder andere Reste R³ substituiert sein kann. Ganz besonders bevorzugt ist R¹ gleich oder verschieden bei jedem Auftreten gewählt aus H oder D.

Bevorzugt sind weiterhin Verbindungen gemäß Formel (1), in denen das Symbol R², welches an die Gruppe X gebunden ist, bei jedem Auftreten gleich oder verschieden ausgewählt ist aus H, geradkettigen Alkylgruppen mit 1 bis 10 C-Atomen oder verzweigten oder cyclischen Alkylgruppen mit 3 bis 10 C-Atomen, wobei jeweils eine oder mehrere nicht benachbarte CH₂-Gruppen durch -R²C=CR²- oder -O- ersetzt sein können und wobei ein oder mehrere H-Atome durch F ersetzt sein können, oder einer monovalenten Aryl- oder Heteroarylgruppe mit 5 bis 16 aromatischen Ringatomen, die mit einem oder mehreren nicht-aromatischen Resten R² substituiert sein kann; dabei können auch zwei Reste R², die in derselben Gruppe X gebunden sind, miteinander ein Ringsystem bilden. Besonders bevorzugt sind in die Reste R² ausgesucht aus geradkettigen Alkylgruppen mit 1 bis 4 C-Atomen oder verzweigten Alkylgruppen mit 3 oder 4 C-Atomen, insbesondere Methylgruppen, oder Phenylgruppen; dabei können zwei oder mehrere Reste R² miteinander ein Ringsystem bilden. Wenn mehrere Reste R² miteinander ein Ringsystem bilden, wird hierdurch eine Spiro-Struktur gebildet. Dies kann insbesondere dann bevorzugt sein, wenn die Reste R² für Phenylgruppernstehen oder wenn zwei Reste R² für Alkylgruppen stehen, die miteinander ein Ringsystem bilden.

Beispiele für bevorzugte Verbindungen gemäß Formel (1) sind die im Folgenden abgebildeten Strukturen (1) bis (10).

| | |
|---|---|
| 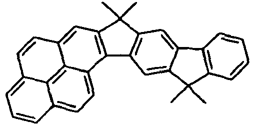 | 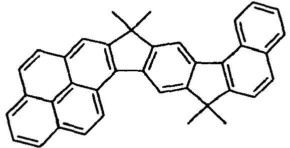 |
| (1) | (2) |
| 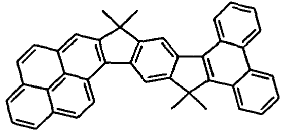 | 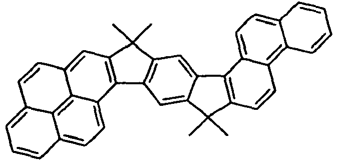 |
| (3) | (4) |
| 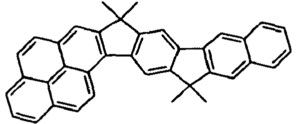 | 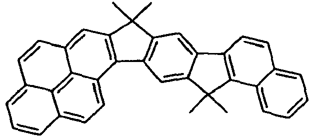 |
| (5) | (6) |
| 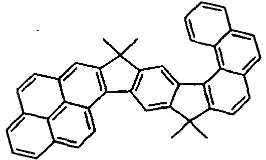 | 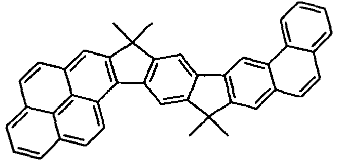 |
| (7) | (8) |
| 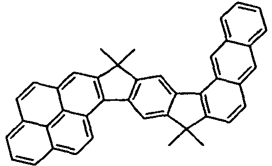 | 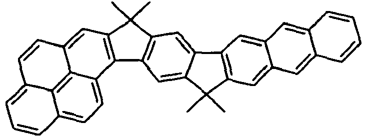 |
| (9) | (10) |

Als blaue Dotanden, welche die oben genannte Bedingung für das HOMO erfüllen, kommen weiterhin beispielsweise die folgenden Verbindungen in Frage:
- Anthracenderivate, beispielsweise Silyl-substituierte Anthracenderivate oder Silylalkinyl-substituierte Anthracenderivate (z. B. gemäß T. Karatsu et al., Organic Electronics 2007, 8, 357-366), Aryl-substituierte Anthracenderivate (z. B. gemäß Y. Kan et al., Synthetic Metals 2004, 141, 245-249) oder Spirobifluoren-substituierte Anthracenderivate (z. B. gemäß D. Gebeyehu et al., Synthetic Metals 2005, 148, 205-211).
- Benzofuranderivate, beispielsweise Alkenyl-substituierte Benzofuranderivate (z. B. gemäß J. R. Hwu et al., Org. Lett. 2005, 7 (8), 1545-1548).
- Imidazophenanthrolinderivate (z. B. gemäß R.-Y. Wang et al., Adv. Funct. Mater. 2005, 15, 1483-1487).
- Pyrenderivate (z. B. gemäß S. L. Tao et al., Adv. Funct. Mater. 2005, 15. 1716-1721).

Als Hostmaterialien für den blauen Dotanden kommen Materialien verschiedener Stoffklassen in Frage. Bevorzugte Hostmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 04/081017), der lochleitenden Verbindungen (z. B. gemäß WO 04/058911), der elektronenleitenden Verbindungen, insbesondere Ketone, Phosphinoxide, Sulfoxide, etc. (z. B. gemäß WO 05/084081 und WO 05/084082), der Atropisomere (z. B. gemäß WO 06/048268), der Boronsäurederivate (z. B. gemäß WO 06/117052) oder der Benzanthracene (z. B. gemäß WO 08/0145239). Besonders bevorzugte Hostmaterialien sind ausgewählt aus den Klassen der Oligoarylene, enthaltend Naphthalin, Anthracen, Benzanthracen, Benzphenanthren und/oder Pyren oder Atropisomere dieser Verbindungen, der Ketone, der Phosphinoxide und der Sulfoxide. Ganz besonders bevorzugte Hostmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind.

Bevorzugte Hostmaterialien sind insbesondere ausgewählt aus Verbindungen der Formel (142),

Ar⁴-(Ar⁵)ₚ-Ar⁶ Formel (142)

wobei Ar⁴, Ar⁵, Ar⁶ bei jedem Auftreten gleich oder verschieden eine Aryl - oder Heteroarylgruppe mit 5 bis 30 aromatischen Ringatomen ist, die mit einem oder mehreren Resten R¹ substituiert sein kann, und R¹ und p dieselbe Bedeutung haben, wie oben beschrieben; dabei gilt, dass die Summe der π-Elektronen in Ar⁴, Ar⁵ und Ar⁶ mindestens 30 beträgt, wenn p = 1 ist, und mindestens 36 beträgt, wenn p = 2 ist, und mindestens 42 beträgt, wenn p = 3 ist.

Besonders bevorzugt steht in den Hostmaterialien der Formel (142) die Gruppe Ar⁵ für Anthracen, welches durch einen oder mehrere Reste R¹ substituiert sein kann, und die Gruppen-Ar⁴ und Ar⁶ sind bevorzugt in 9 - und 10-Position gebunden. Ganz besonders bevorzugt ist mindestens eine der Gruppen Ar⁴ und/oder Ar⁶ eine kondensierte Arylgruppe, ausgewählt aus 1- oder 2-Naphthyl, 2-, 3- oder 9-Phenanthrenyl oder 2-, 3-, 4-, 5-, 6 - oder 7-Benzanthracenyl, welche jeweils durch einen oder mehrere Reste R¹ substituiert sein kann.

Im Folgenden werden die bevorzugten Ausführungsformen für die weiteren emittierenden Schichten und für die weiteren Schichten der OLED ausgeführt.

In den rot und grün emittierenden Schichten können generell alle Materialien verwendet werden, wie sie gemäß dem Stand der Technik verwendet werden.

Im Folgenden werden bevorzugte Ausführungsformen für die phosphoreszierende Verbindung, welche in der phosphoreszierenden Emitterschicht vorhanden ist, ausgeführt.

Als phosphoreszierende Verbindung eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten.

Besonders bevorzugte organische Elektrolumineszenzvorrichtungen enthalten als phosphoreszierende Verbindung mindestens eine Verbindung der Formeln (143) bis (146), 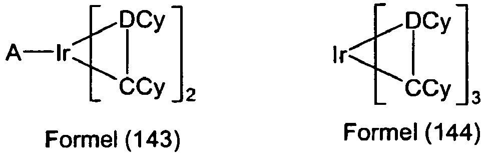 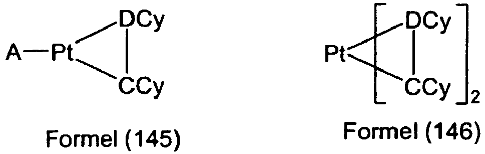 wobei R¹ dieselbe Bedeutung hat, wie oben für Formel (1) beschrieben, und für die weiteren verwendeten Symbole gilt:
- DCy: ist gleich oder verschieden bei jedem Auftreten eine cyclische Gruppe, die mindestens ein Donoratom, bevorzugt Stickstoff, Kohlenstoff in Form eines Carbens oder Phosphor, enthält, über welches die cyclische Gruppe an das Metall gebunden ist, und die wiederum einen oder mehrere Substituenten R¹ tragen kann; die Gruppen DCy und CCy sind über eine kovalente Bindung miteinander verbunden;
- CCy: ist gleich oder verschieden bei jedem Auftreten eine cyclische Gruppe, die ein Kohlenstoffatom enthält, über welches die cyclische Gruppe an das Metall gebunden ist und die wiederum einen oder mehrere Substituenten R¹ tragen kann;
- A: ist gleich oder verschieden bei jedem Auftreten ein monoanionischer, zweizähnig chelatisierender Ligand, bevorzugt ein Diketonatligand.

Dabei kann durch Bildung von Ringsystemen zwischen mehreren Resten R¹ auch eine Brücke zwischen den Gruppen DCy und CCy vorliegen. Weiterhin kann durch Bildung von Ringsystemen zwischen mehreren Resten R¹ auch eine Brücke zwischen zwei oder drei Liganden CCy-DCy bzw. zwischen ein oder zwei Liganden CCy-DCy und dem Liganden A vorliegen, so dass es sich um ein polydentates bzw. polypodales Ligandensystem handelt.

Beispiele der oben beschriebenen Emitter können den Anmeldungen WO 00/70655, WO 01/41512, WO 02/02714, WO 02/15645, EP 1191613, EP 1191612, EP 1191614, WO 04/081017, WO 05/033244, WO 05/042550, WO 05/113563, WO 06/008069, WO 06/061182, WO 06/081973 und der nicht offen gelegten Anmeldung DE 102008027005.9 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Verbindungen verwenden. Insbesondere ist dem Fachmann bekannt, welche phosphoreszierenden Komplexe mit welcher Emissionsfarbe emittieren.

Dabei ist die grün phosphoreszierende Verbindung bevorzugt eine Verbindung der oben genannten Formel (144), insbesondere Tris(phenyl-pyridyl)iridium, welches mit einem oder mehreren Resten R¹ substituiert sein kann.

Als Matrixmaterial für die phosphoreszierende Verbindung kommen verschiedene Materialien in Frage, wie sie gemäß dem Stand der Technik als Matrixmaterialien für phosphoreszierende Verbindungen verwendet werden. Geeignete Matrixmaterialien für den phosphoreszierenden Emitter sind aromatische Ketone, aromatische Phosphinoxide oder aromatische Sulfoxide oder Sulfone, z. B. gemäß WO 04/013080, WO 04/093207, WO 06/005627 oder der nicht offen gelegten Anmeldung DE 102008033943.1, Triarylamine, Carbazolderivate, z. B. CBP (N,N-Biscarbazolylbiphenyl) oder die in WO 05/039246, US 2005/0069729, JP 2004/288381, EP 1205527 oder WO 08/086851 offenbarten Carbazolderivate, Indolocarbazolderivate, z. B. gemäß WO 07/063754 oder WO 08/056746, Azacarbazolderivate, z. B. gemäß EP 1617710, EP 1617711, EP 1731584, JP 2005/347160, bipolare Matrixmaterialien, z. B. gemäß WO 07/137725, Silane, z. B. gemäß WO 05/111172, Azaborole oder Boronester, z. B. gemäß WO 06/117052, Triazinderivate, z. B. gemäß der nicht offen gelegten Anmeldung DE 102008036982.9, WO 07/063754 oder WO 08/056746, Zinkkomplexe, z. B. gemäß EP 652273 oder der nicht offen gelegten Anmeldung DE 102007053771.0, oder Diazasilol- bzw. Tetraazasilol-Derivate, z. B. gemäß der nicht offen gelegten Anmeldung DE 102008056688.8.

Es kann auch Vorteile haben, in einer oder mehreren phosphoreszierenden Emitterschichten eine Mischung aus einem lochleitenden und einem elektronenleitenden Matrixmaterial zu verwenden.

Außer Kathode, Anode und den emittierenden Schichten, die oben beschrieben wurden, kann die organische Elektrolumineszenzvorrichtung noch weitere Schichten enthalten, welche nicht in Abbildung 1 abgebildet sind. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Exzitonenblockierschichten, Ladungserzeugungsschichten (Charge-Generation Layers) und/oder organischen oder anorganischen p/n-Übergängen. Außerdem können auch Zwischenschichten vorhanden sein, insbesondere zwischen einer fluoreszierenden und einer phosphoreszierenden Schicht. Weiterhin kann auch die Verwendung von mehr als drei emittierenden Schichten bevorzugt sein. Weiterhin können die Schichten, insbesondere die Ladungstransportschichten, auch dotiert sein. Die Dotierung der Schichten kann für einen verbesserten Ladungstransport vorteilhaft sein. Es sei aber darauf hingewiesen, dass nicht notwendigerweise jede dieser Schichten vorhanden sein muss und die Wahl der Schichten immer auch von den verwendeten Verbindungen abhängt.

Die Verwendung derartiger Schichten ist dem Fachmann bekannt, und er kann hierfür ohne erfinderisches Zutun alle für derartige Schichten bekannten Materialien gemäß dem Stand der Technik verwenden.

Als Kathode der erfindungsgemäßen Elektrolumineszenzvorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Bei mehrlagigen Strukturen können auch zusätzlich zu den genannten Metallen weitere Metalle verwendet werden, die eine relativ hohe Austrittsarbeit aufweisen, wie z. B. Ag, wobei dann in der Regel Kombinationen der Metalle, wie beispielsweise Ca/Ag oder Ba/Ag verwendet werden. Ebenso bevorzugt sind Metalllegierungen, insbesondere Legierungen aus einem Alkalimetall oder Erdalkalimetall und Silber, besonders bevorzugt eine Legierung aus Mg und Ag. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, CsF, Cs₂CO₃, BaF₂, MgO, NaF, etc.). Die Schichtdicke dieser Schicht beträgt bevorzugt zwischen 0.5 und 5 nm.

Als Anode der erfindungsgemäßen Elektrolumineszenzvorrichtung sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Dabei muss mindestens eine der Elektroden transparent sein, um die Auskopplung von Licht zu ermöglichen. Ein bevorzugter Aufbau verwendet eine transparente Anode. Bevorzugte Anodenmaterialien sind hier leitfähige gemischte Metalloxide. Besonders bevorzugt sind Indium-Zinn-Oxid (ITO) oder Indium-Zink Oxid (IZO). Bevorzugt sind weiterhin leitfähige, dotierte organische Materialien, insbesondere leitfähige dotierte Polymere.

Die Vorrichtung wird entsprechend (je nach Anwendung) strukturiert, kontaktiert und schließlich hermetisch versiegelt, da sich die Lebensdauer derartiger Vorrichtungen bei Anwesenheit von Wasser und/oder Luft drastisch verkürzt.

Es können generell alle weiteren Materialien, wie sie gemäß dem Stand der Technik in organischen Elektrolumineszenzvorrichtungen eingesetzt werden, auch in Kombination mit der erfindungsgemäßen blauen Emitterschicht in der weiß emittierenden OLED eingesetzt werden.

Geeignete Ladungstransportmaterialien, wie sie in der Lochinjektions- bzw. Lochtransportschicht oder in der Elektronentransportschicht der erfindungsgemäßen organischen Elektrolumineszenzvorrichtung verwendet werden können, sind beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Beispiele für bevorzugte Lochtransportmaterialien, die in einer Lochtransport- oder Lochinjektionsschicht in der erfindungsgemäßen Elektrolumineszenzvorrichtung verwendet werden können, sind Indenofluorenamine und Derivate (z. B. gemäß WO 06/122630 oder WO 06/100896), die in EP 1661888 offenbarten Aminderivate, Hexaazatriphenylenderivate (z. B. gemäß WO 01/049806), Aminderivate mit kondensierten Aromaten (z. B. gemäß US 5,061,569), die in WO 95/09147 offenbarten Aminderivate, Monobenzoindenofluorenamine (z. B. gemäß WO 08/006449) oder Dibenzoindenofluorenamine (z. B. gemäß WO 07/140847). Weiterhin geeignete Lochtransport- und Lochinjektionsmaterialien sind Derivate der oben abgebildeten Verbindungen, wie sie in JP 2001/226331, EP 676461, EP 650955, WO 01/049806, US 4780536, WO 98/30071, EP 891121, EP 1661888, JP 2006/253445, EP 650955, WO 06/073054 und US 5061569 offenbart werden.

Geeignete Lochtransport- oder Lochinjektionsmaterialien sind weiterhin beispielsweise die in der folgenden Tabelle aufgeführten Materialien.

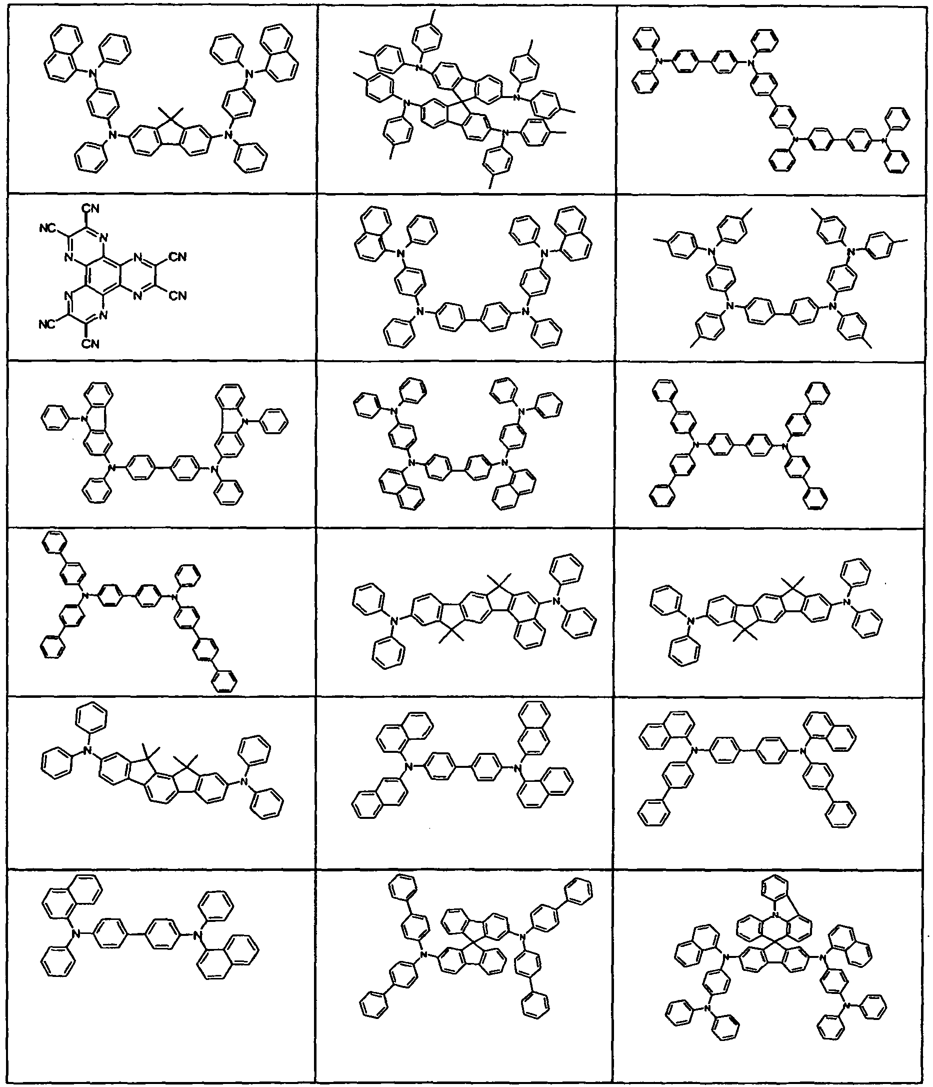

Als Materialien für die Elektronentransportschicht können alle Materialien verwendet werden, wie sie gemäß dem Stand der Technik als Elektronentransportmaterialien in der Elektronentransportschicht verwendet werden. Insbesondere eignen sich Aluminiumkomplexe, beispielsweise Alq₃, Zirkoniumkomplexe, beispielsweise Zrq₄, Benzimidazolderviate, Triazinderivate oder aromatische Ketone. Geeignete Materialien sind beispielsweise die in der folgenden Tabelle aufgeführten Materialien. Weiterhin geeignete Materialien sind Derivate der oben abgebildeten Verbindungen, wie sie in JP 2000/053957, WO 03/060956, WO 04/028217 und WO 04/080975 offenbart werden.

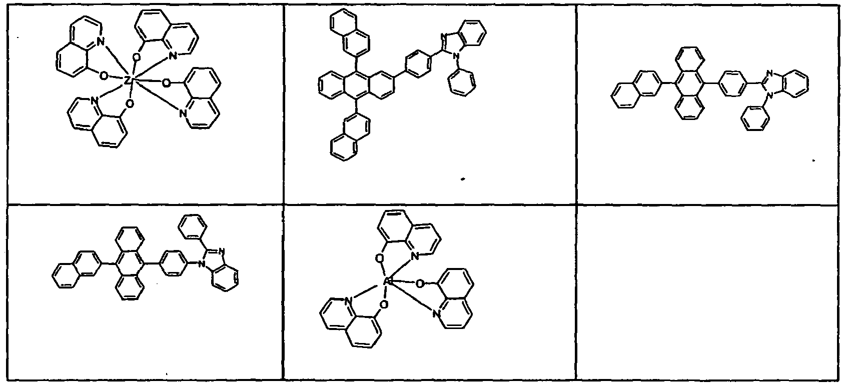

Weiterhin ist es möglich, dass die Elektronentransportschicht dotiert ist. Geeignete Dotanden sind Alkalimetalle oder Alkalimetallverbindungen, wie zum Beispiel Liq (Lithiumchinolinat). In einer bevorzugten Ausführungsform der Erfindung ist die Elektronentransportschicht insbesondere dann dotiert, wenn das Elektronentransportmaterial ein Benzimidazolderivat oder ein Triazinderivat ist. Der bevorzugte Dotand ist dann Liq.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es sei jedoch angemerkt, dass der Druck auch noch geringer sein kann, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Amold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig. Hohe Löslichkeit lässt sich durch geeignete Substitution der Verbindungen erreichen. Dabei können nicht nur Lösungen aus einzelnen Materialien aufgebracht werden, sondern auch Lösungen, die mehrere Verbindungen enthalten, beispielsweise Matrixmaterialien und Dotanden.

Die organische Elektrolumineszenzvorrichtung kann auch hergestellt werden, indem eine oder mehrere Schichten aus Lösung aufgebracht werden und eine oder mehrere andere Schichten aufgedampft werden.

Diese Verfahren sind dem Fachmann generell bekannt und können von ihm ohne erfinderisches Zutun auf die erfindungsgemäßen organischen Elektrolumineszenzvorrichtungen angewandt werden.

Die erfindungsgemäße organische Elektrolumineszenzvorrichtung weist folgende überraschende Vorteile gegenüber dem Stand der Technik auf:
1. Die erfindungsgemäße organische Elektrolumineszenzvorrichtung weist eine sehr gute Lebensdauer auf. Insbesondere ist diese Lebensdauer deutlich verbessert gegenüber einer OLED, welche aminhaltige blau fluroeszierende Dotanden enthält.
2. Die erfindungsgemäße OLED weist eine verbesserte Betriebsspannung und Leistungseffizienz gegenüber einer OLED, welche aminhaltige blau fluoreszierende Dotanden enthält, auf.

Die Erfindung wird durch die nachfolgenden Beispiele genauer beschrieben, ohne sie dadurch einschränken zu wollen. Der Fachmann kann, ohne erfinderisch tätig zu werden, die Erfindung im gesamten offenbarten Bereich ausführen und so weitere erfindungsgemäße organische Elektrolumineszenzvorrichtungen herstellen.

### Beispiele:

### Beispiel 1: Bestimmung von HOMO, LUMO und Energielücke aus Cyclovoltammetrie und Absortionsspektrum

Die Bestimmung der HOMO- und LUMO-Werte sowie der Energielücke im Sinne der vorliegenden Erfindung erfolgt nach den im Folgenden beschriebenen allgemeinen Verfahren:

Der HOMO-Wert ergibt sich aus dem Oxidationspotential, das mittels Cyclovoltammetrie (CV) bei Raumtemperatur gemessen wird. Als Messgerät hierzu wird ein ECO Autolab System mit Metrohm 663 VA Stand verwendet. Die Arbeitselektrode ist eine Goldelektrode,. die Referenzelektrode Ag/AgCl, der Zwischenelektrolyt KCI (3 mol/l) und die Hilfselektrode Platin.

Für die Messung wird zunächst eine 0.11 M Leitsalzlösung aus Tetrabutylammoniumhexafluorophosphat (NH₄PF₆) in Dichlormethan hergestellt, in die Messzelle eingefüllt und 5 min entgast. Anschließend werden zwei Messzyklen mit folgenden Parametern durchgeführt:
Messtechnik: CV
Initial purge time: 300 s
Cleaning Potential: -1 V
Cleaning Time: 10 s
Deposition Potential: -0.2 V
Deposition Time: 10 s
Start Potential: -0.2 V
End Potential: 1.6 V
Voltage Step: 6 mV
Sweep Rate: 50 mV/s

Anschließend wird die Leitsalzlösung mit 1 ml der Probenlösung (10 mg der zu messenden Substanz in 1 ml Dichlormethan) versetzt und erneut 5 min entgast. Anschließend werden fünf weitere Messzyklen gefahren, von denen die letzten 3 zur Auswertung aufgezeichnet werden. Dabei werden dieselben Parameter eingestellt, wie oben beschrieben.

Anschließend wird die Lösung mit 0.1 ml Ferrocenlösung (100 mg Ferrocen in 1 ml Dichlormethan) versetzt, 1 min entgast, und ein Messzyklus mit folgenden Parametern durchgeführt:
Messtechnik: CV
Initial purge time: 60 s
Cleaning Potential: -1 V
Cleaning Time: 10 s
Deposition Potential: -0.2 V
Deposition Time: 10 s
Start Potential: -0.2 V
End Potential: 1.6 V
Voltage Step: 6 mV
Sweep Rate: 50 mV/s

Zur Auswertung wird für die Probenlösung und die mit Ferrocenlösung versetzte Lösung jeweils der Mittelwert aus den Spannungen des ersten Oxidationsmaximums aus den Hinkurven und des zugehörigen Reduktionsmaximums aus den Rückkurven genommen (V_{P} und V_{F}), wobei als Spannung jeweils die Spannung gegen Ferrocen verwendet wird. Der HOMO-Wert der zu untersuchenden Substanz E_{HOMO} ergibt sich als E_{HOMO} = -[e·(V_{P} - V_{F}) + 4.8 eV], wobei e die Elementarladung darstellt.

Es ist zu beachten, dass im Einzelfall gegebenenfalls sinnvolle Abwandlungen der Messmethode vorgenommen werden müssen, z. B. wenn sich die zu untersuchende Substanz in Dichlormethan nicht lösen lässt oder wenn es zu Zersetzung der Substanz während der Messung kommt. Sollte eine sinnvolle Messung mittels CV mit der oben genannten Methode nicht möglich sein, wird die HOMO-Energie über Photoelektronen-Spektroskopie mittels Model AC-2 Photoelectron Spectrometer von Riken Keiki Co. Ltd. (http://www.rikenkeiki.com/pages/AC2.htm) bestimmt werden, wobei zu beachten ist, dass die erhaltenen Werte dabei typischerweise um 0.3 eV tiefer liegen, als die mit CV gemessenen. Unter dem HOMO-Wert im Sinne dieses Patents ist dann der Wert aus Riken AC2 + 0.3 eV zu verstehen.

Weiterhin lassen sich sowohl mit der beschriebenen CV Methode wie auch mit der beschriebenen Photoelektronen-Spektroskopie HOMO-Werte, die tiefer als -6 eV liegen, nicht verlässlich messen. In diesem Fall werden die HOMO-Werte aus quantenchemischer Rechnung mittels Dichtefunktional-Theorie (DFT) ermittelt. Dies erfolgt über die kommerziell erhältliche Software Gaussian 03W (Gaussian Inc.) mit der Methode B3PW91 / 6-31G(d). Die Normierung der berechneten Werte auf CV-Werte wird über einen Abgleich mit aus CV messbaren Materialien erreicht. Hierzu werden die HOMO-Werte einer Reihe von Materialien mit der CV Methode gemessen und ebenso berechnet. Die berechneten Werte werden dann mittels der gemessenen kalibriert, dieser Kalibrierfaktor wird für alle weiteren Berechnungen verwendet. Auf diese Weise lassen sich HOMO-Werte berechnen, die sehr gut denen entsprechen, die man über CV messen würde. Sollte sich der HOMO-Wert einer bestimmten Substanz nicht über CV oder Riken AC2 wie oben beschrieben messen lassen, ist unter dem HOMO-Wert im Sinne dieses Patents daher der Wert zu verstehen, der entsprechend der Beschreibung durch eine auf CV kalibrierte DFT-Rechnung, wie oben beschrieben, erhalten wird. Beispiele für auf diese Weise berechnetete Werte einiger gäninger organischer Materialien sind: NPB = (HOMO -5.16 eV, LUMO -2.28 eV); TCTA (HOMO -5.33 eV, LUMO -2.20 eV); TPBI (HOMO -6.26 eV, LUMO -2.48 eV). Diese Werte können für die Kalibrierung der Rechenmethode verwendet werden.

Die Energielücke wird bestimmt aus der Absorptionskante des Absorptionsspektrums gemessen an einem Film mit Schichtdicke 50 nm. Die Absorptionskante ist dabei definiert als die Wellenlänge, die man erhält, wenn man im Absorptionsspektrum an die langwelligste abfallende Flanke an ihrer steilsten Stelle eine Gerade anfittet und den Wert ermittelt, bei dem diese Gerade die Wellenlängeachse, d. h.den Absorptionswert = 0, schneidet

Der LUMO Wert wird durch Addition der Energielücke auf den oben beschriebenen HOMO-Wert erhalten.

### Beispiel 2: Synthese von 1,1-Dimethylbenzindeno-1,1-dimethyl-indeno-[a]pyren

### a) 2-Chlor-5-pyren-1-yl-terephthalsäurediethylester

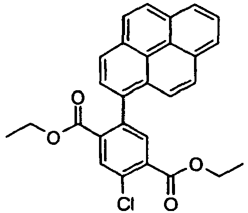

28.9 g (103 mmol) Brompyren werden in 275 mL trockenem THF gelöst, auf -75 °C abgekühlt und bei dieser Temperatur 52 mL (104 mmol) einer 2 M Lösung von n-Buthyllithium zugetropft. Die gelbe Suspension wird 1 h bei -75 °C gerührt und dann 17.5 mL (155 mmol) Trimethylborat zugetropft. Nach Erwärmen auf RT werden 34.5 g (103 mmol) Chlorbromterephthalsäurediethylester, 22 g (206 mmol) Na₂CO₃, 1.2 g (1.03 mmol) Tetrakis(triphenylphosphin)-palladium(O), 140 mL H₂O, 280 mL Toluol und 140 mL EtOH zugegeben und das Gemisch für 2 h zum Sieden erhitzt. Nach Abtrennen der organischen Phase, zweimaligem Waschen mit Wasser und Trocknen über Na₂SO₄ wird das Lösungsmittel im Vakuum entfernt und das verbleibende Öl in Heptan zur Kristallisation gebracht. Zweimalige Umkristallisation liefert das Produkt in Form eines farblosen Feststoffes (33 g, 70 %) und einer Reinheit von >98 %, was in dieser Form in die Folgereaktion eingesetzt wird.

### b) 2-Naphthalin-1-yl-5-pyren-1-yl-terephthalsäurediethylester

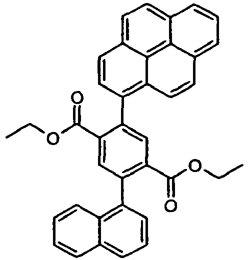

43.5 g (90 mmol) 2-Chlor-5-pyren-1-yl-terephthalsäurediethylester, 21.5 g (120 mmol) 1-Naphthylboronsäure und 58.1 g Cs₂CO₃ werden in 230 mL trockenem Dioxan vorgelegt und 30 min mit N₂ gesättigt. Danach erfolgt Zugabe von 2.7 mL einer 1.0 M Lösung von Tri-*tert*-butylphosphin in Toluol, gefolgt von 300 mg (1.3 mmol) Pd(OAc)₂. Das Gemisch wird für 4 h zum Sieden erhitzt, mit Wasser und EtOH erweitert, der Niederschlag abgesaugt, mit Wasser und EtOH gewaschen und getrocknet. Der Feststoff wird dreimal aus Dioxan umkristallisiert und weist danach gemäss ¹H-NMR eine Reinheit von >99% auf. Die Ausbeute beträgt 44,2 g (90%) an farblosem Feststoff.

In Analogie zum oben beschriebenen Verfahren wird die folgende Verbindung hergestellt (Beispiel 3b).

| Bsp. | Struktur | Ausbeute (%) |
|---|---|---|
| 3b | 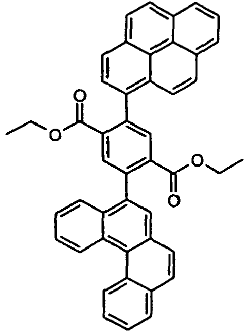 | 67 |

### c) 2-[4-(1-Hydroxy-1-methyl-ethyl)-2-naphthalin-1-yl-5-pyren-1-yl-phenyl]-propan-2-ol

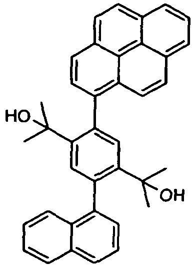

30 g (55 mol) 2-Naphthalin-1-yl-5-pyren-1-yl-terephthalsäurediethylester werden in 270 mL trockenem THF gelöst, bei 5 °C 110 mL (330 mmol) einer 3 M Methylmagnesiumchlorid Lösung in THF zugetropft und das Gemisch für 12 h bei RT gerührt. Nach Unterbrechung der Reaktion durch Zugabe von 180 mL 25 %iger Essigsäure wird extraktiv mit Essigsäureethylester/Wasser aufgearbeitet, über Na₂SO₄ getrocknet und einrotiert. Nach Umkristallisation aus EtOH/Toluol verbleiben 26.3 g (92 %) farbloser Feststoff, der nach ¹H-NMR eine Reinheit von >98 % aufweist.

In Analogie zum oben beschriebenen Verfahren wird die folgende Verbindung hergestellt (Beispiel 3c).

| Bsp. | Struktur | Ausbeute (%) |
|---|---|---|
| 3c | 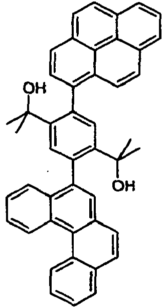 | 80 |

### d) 1,1-Dimethylbenzindeno-1,1-dimethylindeno[a]pyren (BD1)

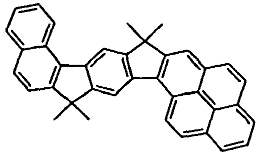

26.3 g (50.5 mmol) 2-[4-(1-Hydroxy-1-methyl-ethyl)-2-naphthalin-1-yl-5-pyren-1-yl-phenyl]-propan-2-ol werden in 750 mL Dichlormethan gelöst, 45 mL Methansulfonsäure in 70 g Polyphosphorsäure bei -20 °C zugetropft und 1 h bei dieser Temperatur gerührt. Nach vollständiger Umsetzung werden 400 mL EtOH zugetropft, 1 h zum Sieden erhitzt und der gelbe Feststoff abfiltriert. Nach viermaliger Umkristallisation aus NMP und zweimaliger Sublimation im Vakuum (p = 1 x 10⁻⁵ mbar, T = 340 °C) erhält man ein gelbes Pulver mit einer Reinheit >99.9 % (16 g, 65 %).

In Analogie zum oben beschriebenen Verfahren wird die folgende Verbindung hergestellt (Beispiel 3d, BD2).

| Bsp. | Struktur | Ausbeute (%) |
|---|---|---|
| 3d | 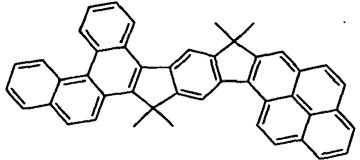 | 15 |

### Beispiel 4: Herstellung der Elektrolumineszenzvorrichtungen

Erfindungsgemäße Elektrolumineszenzvorrichtungen können, wie beispielsweise in WO 05/003253 beschrieben, hergestellt werden.

Die Strukturen der verwendeten Materialien sind der Übersichtlichkeit halber im Folgenden abgebildet.

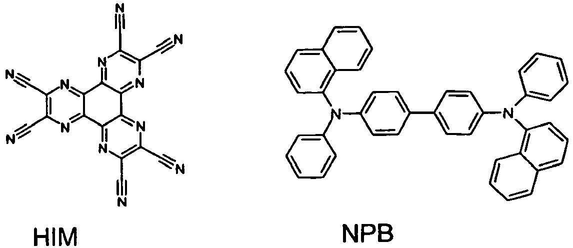

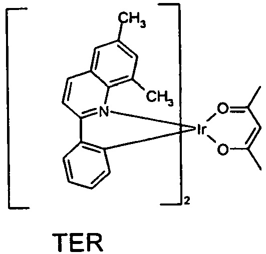

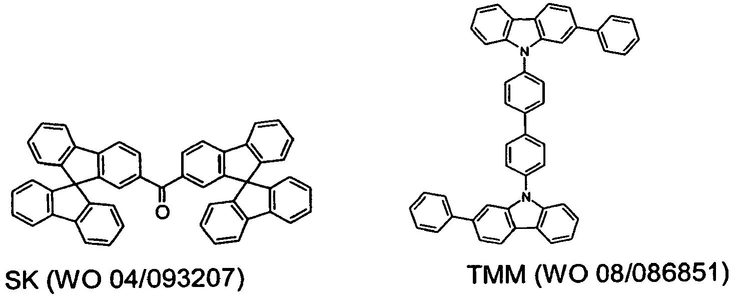

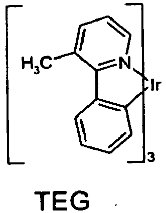

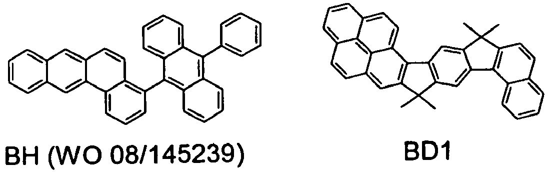

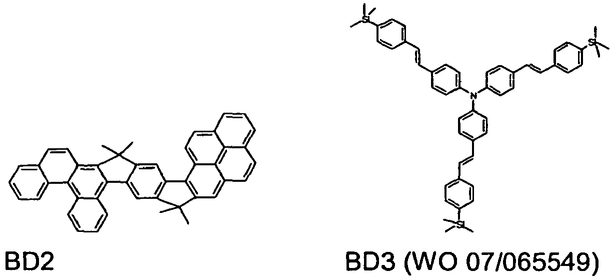

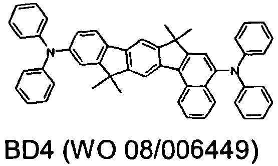

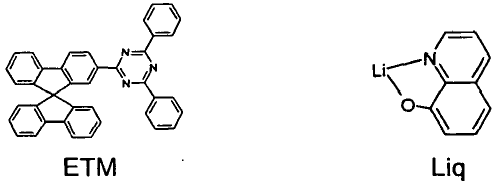

Diese noch nicht optimierten OLEDs werden standardmäßig charakterisiert; hierfür werden die Elektrolumineszenzspektren und Farbkoordinaten (gemäß CIE 1931), die Effizienz (gemessen in cd/A) in Abhängigkeit von der Helligkeit, die Betriebsspannung, berechnet aus Strom-Spannungs-Leuchtdichte-Kennlinien (IUL-Kennlinien), und die Lebensdauer bestimmt. Die erhaltenen Ergebnisse sind in Tabelle 1 zusammengefasst.

Im Folgenden werden die Ergebnisse verschiedener weißer OLEDs gegenübergestellt. Die blauen Dotanden werden dabei so gewählt, dass jeweils der Dotand aus dem erfindungsgemäßen Beispiel (BD1 oder BD2) in einer monochrom blauen OLED die gleiche Farbkoordinate ergibt wie der Dotand aus dem Vergleichsbeispiel (BD3 oder BD4). Die monochrome OLED wird dabei durch folgenden Schichtaufbau realisiert: 20 nm HIM, 20 nm NPB, 25 nm BH dotiert mit 2.5% BD, 25 nm ETM dotiert mit 50% Liq, 100 nm Al. BD1 und BD3 führen dabei zu blauer Emission mit Farbkoordinaten CIE1931 0.14/0.11, BD2 und BD4 zu CIE1931 0.14/0.16.

### Beispiel 5:

Erfindungsgemäßes Beispiel 5 wird durch folgenden Schichtaufbau realisiert: 20 nm HIM, 40 nm NPB dotiert mit 7% TER, 8 nm Mischschicht, bestehend aus 80% TMM, 10% SK und 10% TEG, 25 nm BH dotiert mit 2.5% BD1, 5 nm SK, 25 nm ETM dotiert mit 50% Liq, 100 nm Al.

### Beispiel 6:

Erfindungsgemäßes Beispiel 6 wird durch folgenden Schichtaufbau realisiert: 20 nm HIM, 40 nm NPB dotiert mit 7% TER, 8 nm Mischschicht, bestehend aus 80% TMM, 10% SK und 10% TEG, 25 nm BH dotiert mit 2.5% BD2, 5 nm SK, 25 nm ETM dotiert mit 50% Liq, 100 nm Al.

### Beispiel 7 (Vergleich):

Vergleichsbeispiel 7 wird durch folgenden Schichtaufbau realisiert: 20 nm HIM, 40 nm NPB dotiert mit 7% TER, 8 nm Mischschicht, bestehend aus 80% TMM, 10% SK und 10% TEG, 25 nm BH dotiert mit 2.5% BD3, 5 nm SK, 25 nm ETM dotiert mit 50% Liq, 100 nm Al.

### Beispiel 8 (Vergleich):

Vergleichsbeispiel 8 wird durch folgenden Schichtaufbau realisiert: 20 nm HIM, 40 nm NPB dotiert mit 7% TER, 8 nm Mischschicht, bestehend aus 80% TMM, 10% SK und 10% TEG, 25 nm BH dotiert mit 2.5% BD4, 5 nm SK, 25 nm ETM dotiert mit 50% Liq, 100 nm Al.

Beispiele 5 und 7, sowie Beispiele 6 und 8 ergeben dabei jeweils ähnliche Farbkoordinaten, sodass sie Emissionsdaten gut miteinander verglichen werden können. Beispiele 5 und 7 weisen bläulich weiße Emission auf, Beispiele 6 und 8 gelblich weiße Emission. Wie in der nicht offen gelegten Anmeldung DE 102008063490.5 dargestellt, können durch Variation z. B. der Konzentrationsverhältnisse und Schichtdicke der grünen Emitterschicht weitere Farbkoordinaten, z.B. CIE 0.28/0.29 oder CIE 0.45/0.41 realisiert werden. Die erfindungsgemäßen OLEDs weisen auch in diesem Fall analog zu den gezeigten Beispielen verbesserte Emissionseigenschaften gegenüber den Vergleichs-OLEDs auf.

Der Vergleich der Emissionsdaten von Beispiel 5 mit Vergleichsbeispiel 7 bzw. Beispiel 6 mit Vergleichsbeispiel 8 zeigt, dass die erfindungsgemäßen OLEDs sowohl eine verbesserte Leistungseffizienz als auch eine verbesserte Betriebslebensdauer aufweisen.

**Tabelle 1: Device-Ergebnisse**

| Bsp. | Blauer Dotand | HOMO blauer Dotand | Effizienz [lm/W] bei 4000 cd/m² | Spannung [V] bei 4000 cd/m² | CIE x/y bei 4000 cd/m² | Lebensdauer 50% [h], Anfangshelligkeit 4000 cd/m² |
|---|---|---|---|---|---|---|
| 5 | BD1 | -5.4 eV | 7.9 | 5.2 | 0.3110.32 | 1100 |
| 6 | BD2 | -5.35 eV | 10 | 5.0 | 0.37/0.36 | 1100 |
| 7 Vgl. | BD3 | -5.1 eV | 7.1 | 5.5 | 0.31/0.31 | 750 |
| 8 Vgl. | BD4 | -5.1 eV | 9.5 | 5.2 | 0.3710.37 | 900 |

## Patentansprüche

1. Organische Elektrolumineszenzvorrichtung, enthaltend in dieser Reihenfolge eine Anode, eine erste Emitterschicht, eine zweite Emitterschicht, welches eine blau emittierende Schicht ist, wobei die blau emittierende Schicht ein Hostmaterial in einem Anteil von 90 - 99.9 Vol.-% und einen Dotanden in einem Anteil von 0.1 - 10 Vol.-% enthält, und eine Kathode, **dadurch gekennzeichnet, dass** der Dotand ein HOMO, gemessen wie in Beispiel 1 der Beschreibung angegeben, von kleiner als -5.2 eV aufweist.

2. Organische Elektrolumineszenzvorrichtung nach Anspruch 1,
**dadurch gekennzeichnet, dass** die Vorrichtung genau zwei emittierende Schichten aufweist, wobei die Emitterschicht auf Anodenseite eine gelb oder orange emittierende Emitterschicht ist und wobei es sich bevorzugt um eine phosphoreszierende Emitterschicht handelt.

3. Organische Elektrolumineszenzvorrichtung nach Anspruch 1,
**dadurch gekennzeichnet, dass** die Vorrichtung mindestens drei emittierende Schichten aufweist, wobei eine dieser Schichten eine rot oder orange emittierende Emitterschicht und eine der Schichten eine grün emittierende Emitterschicht ist und die rot oder orange emittierende Schicht auf Anodenseite und die grün emittierende Schicht zwischen der rot emittierende Schicht und der blau emittierende Schicht liegt, wobei es sich bevorzugt bei der rot oder orange emittierende Schicht und/oder bei der grün emittierende Schicht um phosphoreszierende Schichten handelt.

4. Organische Elektrolumineszenzvorrichtung nach einem oder mehreren der Ansprüche 1 bis 3, **dadurch gekennzeichnet, dass** es sich bei der blau emittierenden Schicht um eine fluoreszierende Schicht handelt.

5. Organische Elektrolumineszenzvorrichtung nach einem oder mehreren der Ansprüche 1 bis 4, **dadurch gekennzeichnet, dass** der blaue Dotand ein HOMO (highest occupied molecular orbital), gemessen wie in Beispiel 1 der Beschreibung angegeben, von kleiner als -5.3 eV, bevorzugt kleiner als -5.4 eV aufweist.

6. Organische Elektrolumineszenzvorrichtung nach einem oder mehreren der Ansprüche 1 bis 5, **dadurch gekennzeichnet, dass** der blaue Dotand ein LUMO (lowest unoccupied molecular orbital), gemessen wie in Beispiel 1 der Beschreibung angegeben, von kleiner als -2.3 eV aufweist, bevorzugt von kleiner als -2.5 eV.

7. Organische Elektrolumineszenzvorrichtung nach einem oder mehreren der Ansprüche 1 bis 6, **dadurch gekennzeichnet, dass** der blaue Dotand in einer Konzentration von 0.2 - 7 Vol.-% in der blau emittierenden Schicht vorliegt, bevorzugt von 0.5 - 5 Vol.-%, besonders bevorzugt von 0.8 - 3 Vol.-%.

8. Organische Elektrolumineszenzvorrichtung nach einem oder mehreren der Ansprüche 1 bis 7, **dadurch gekennzeichnet, dass** der blaue Dotand eine Verbindung gemäß der folgenden Formel (1) ist, 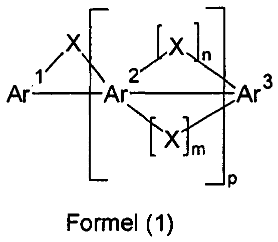 wobei für die verwendeten Symbole und Indizes gilt:
Ar¹, Ar², Ar³ ist bei jedem Auftreten gleich oder verschieden eine Aryl- oder Heteroarylgruppe mit 5 bis 30 aromatischen Ringatomen, die mit einem oder mehreren Resten R¹ substituiert sein kann;
X ist bei jedem Auftreten gleich oder verschieden eine Gruppe, ausgewählt aus BR², C(R²)₂, Si(R²)₂, C=O, C=NR², C=C(R²)₂, O, S, S=O, SO₂, NR², PR², P(=O)R² oder P(=S)R²;
R¹, R² ist bei jedem Auftreten gleich oder verschieden H, D, F, Cl, Br, I, C(=O)Ar⁴, P(=O)(Ar⁴)₂, S(=O)Ar⁴, S(=O)₂Ar⁴, CR²=CR²Ar⁴, CHO, CR³=C(R³)₂, CN, NO₂, Si(R³)₃, B(OR³)₂, B(R³)₂, B(N(R³)₂)₂, OSO₂R³, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R³ substituiert sein kann, wobei jeweils eine oder mehrere nicht benachbarte CH₂-Gruppen durch R³C=CR³, C≡C, Si(R³)₂, Ge(R³)₂, Sn(R³)₂, C=O, C=S, C=Se, C=NR³, P(=O)R³, SO, SO₂, NR³, O, S oder CONR³ ersetzt sein können und wobei ein oder mehrere H-Atome durch F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R³ substituiert sein kann, oder eine Kombination dieser Systeme; dabei können zwei oder mehrere Substituenten R¹ bzw. R² auch miteinander ein mono- oder polycyclisches, aliphatisches oder aromatisches Ringsystem bilden;
R³ ist bei jedem Auftreten gleich oder verschieden H, D oder ein aliphatischer oder aromatischer Kohlenwasserstoffrest mit 1 bis 20 C-Atomen;
Ar⁴ ist bei jedem Auftreten gleich oder verschieden ein aromatisches oder heteroaromatisches Ringsystem mit 5-30 aromatischen Ringatomen, das mit einem oder mehreren nicht-aromatischen Resten R¹ substituiert sein kann; dabei können auch zwei Reste Ar am selben Stickstoff- oder Phosphoratom durch eine Einfachbindung oder eine Brücke X miteinander verknüpft sein;
m, n sind 0 oder 1, mit der Maßgabe, dass m + n = 1 ist;
p ist 1, 2, 3, 4, 5 oder 6;
dabei bilden Ar¹, Ar² und X zusammen einen Fünfring oder einen Sechsring und Ar², Ar³ und X bilden zusammen einen Fünfring oder einen Sechsring;
dabei beträgt die Summe aller π-Elektronen der Gruppen Ar¹, Ar² und Ar³ bevorzugt mindestens 28, wenn p = 1 ist, und mindestens 34, wenn p = 2 ist, und mindestens 40, wenn p = 3 ist, und mindestens 46, wenn p = 4 ist und mindestens 52, wenn p = 5 ist und mindestens 58, wenn p = 6 ist.

9. Organische Elektrolumineszenzvorrichtung nach einem oder mehreren der Ansprüche 1 bis 8, **dadurch gekennzeichnet, dass** die Symbole Ar¹, Ar² und Ar³ gleich oder verschieden bei jedem Auftreten für eine Aryl- oder Heteroarylgruppe mit 5 bis 22 aromatischen Ringatomen, insbesondere mit 5 bis 18 aromatischen Ringatomen, stehen, insbesondere ausgewählt aus der Gruppe bestehend aus Benzol, Naphthalin, Anthracen, Phenanthren, Fluoranthen, Naphthacen, Benzanthracen, Chrysen, Pyren, Benzfluoranthen, Triphenylen, Perylen, Dibenzanthracen, Benzpyren, Picen, Pentacen, Pentaphen, Benzphenanthren, Pyridin, Pyrazin, Pyrimidin, Pyridazin, Chinolin, Isochinolin, Phenanthrolin, Acridin.

10. Organische Elektrolumineszenzvorrichtung nach einem oder mehreren der Ansprüche 1 bis 9, **dadurch gekennzeichnet, dass** die Gruppen Ar¹ und Ar³, welche mit Ar² einen Fünfring bilden, die im Folgenden aufgeführten Gruppen der Formeln (2) bis (85) sind, welche jeweils durch einen oder mehrere Reste R¹ substituiert sein können und dass die Gruppen Ar² die im Folgenden aufgeführten Gruppen der Formeln (86) bis (110) sind, welche jeweils durch einen oder mehrere Reste R¹ substituiert sein können; dabei steht das Symbol * für die Position der Verknüpfung von Ar¹ mit Ar² bzw. Ar² mit Ar³ und das Symbol # steht für die Position der Verknüpfung mit X:

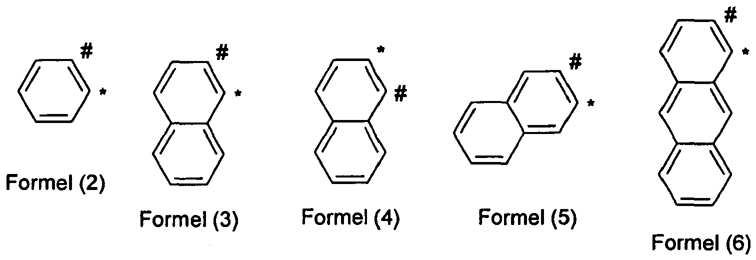

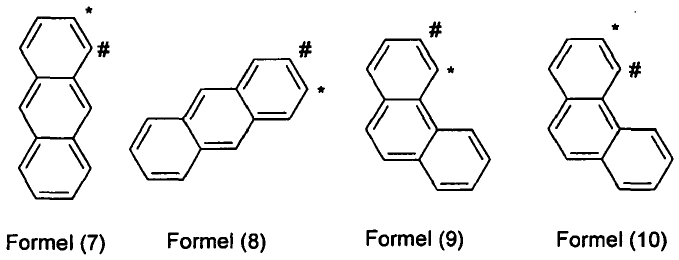

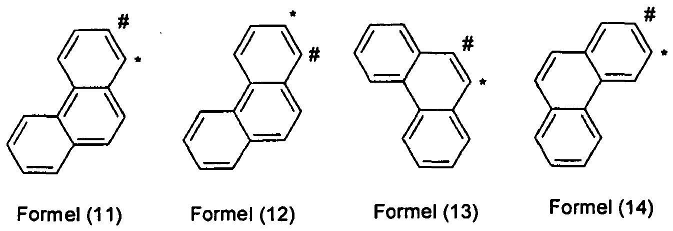

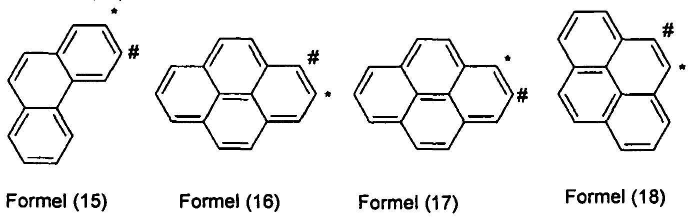

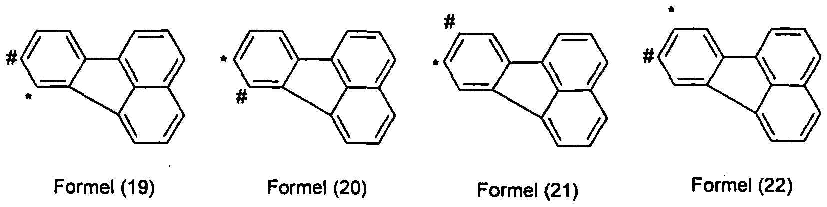

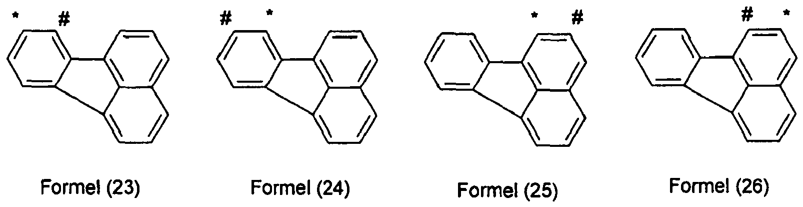

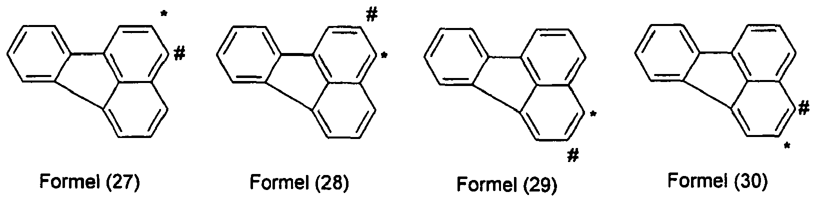

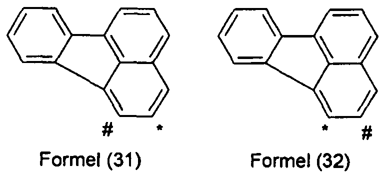

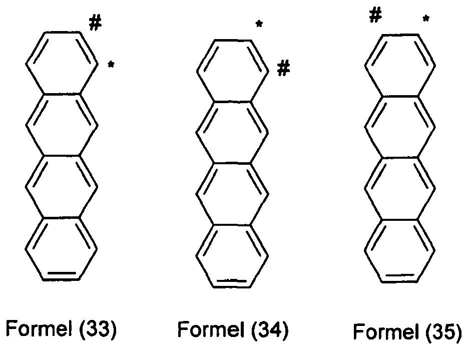

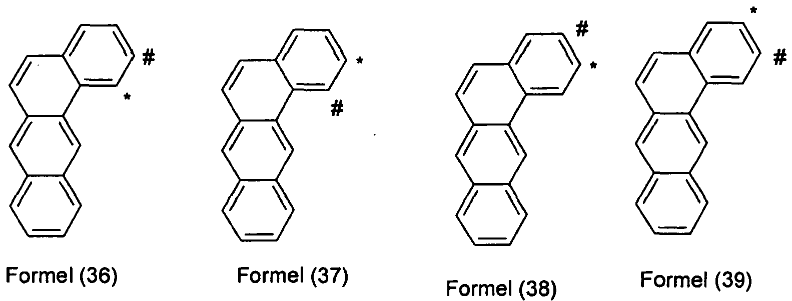

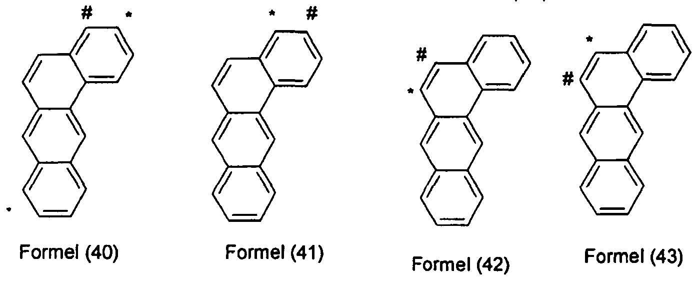

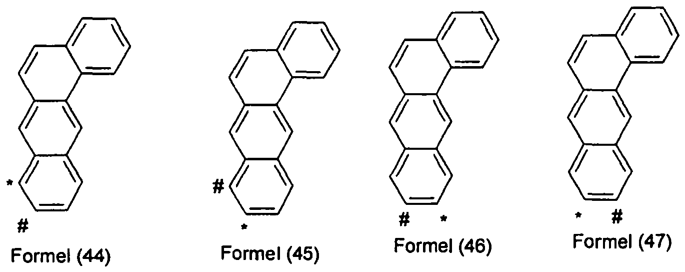

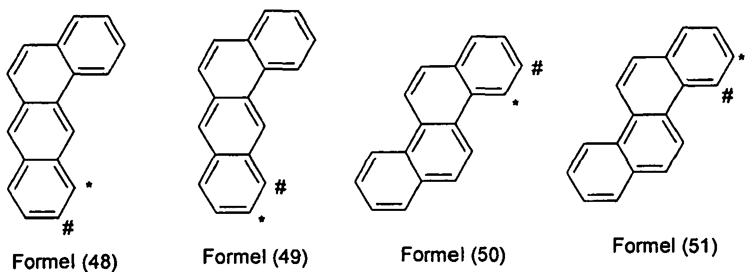

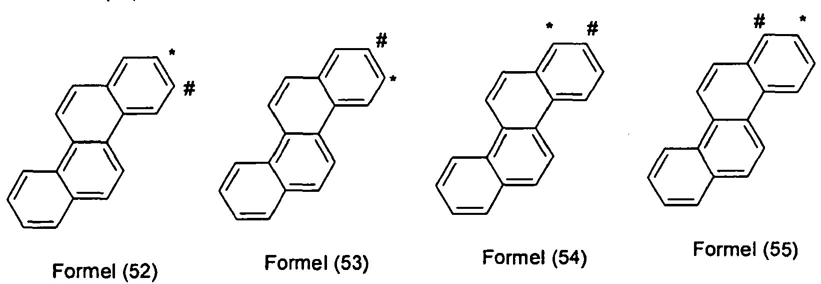

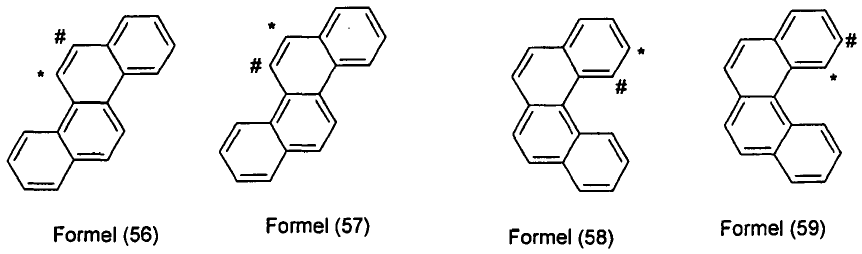

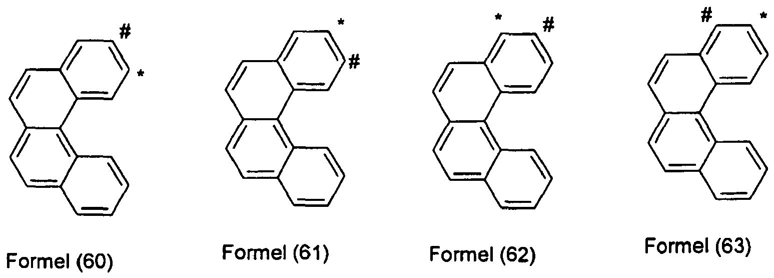

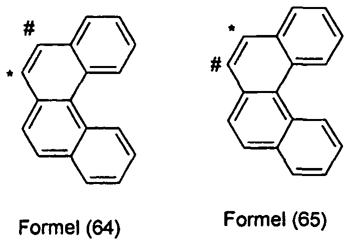

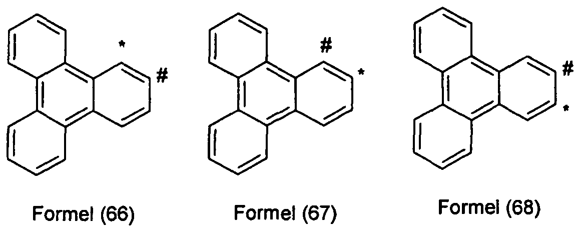

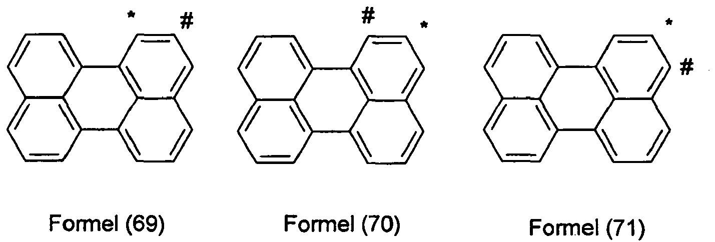

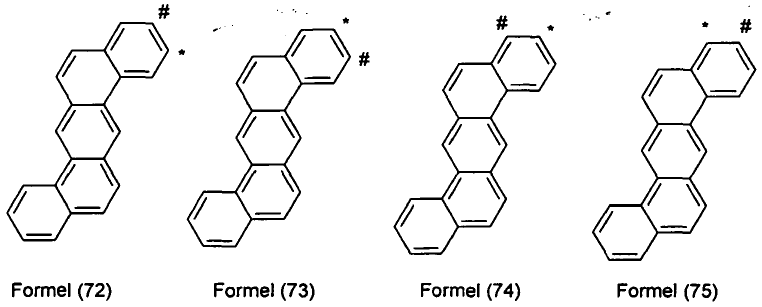

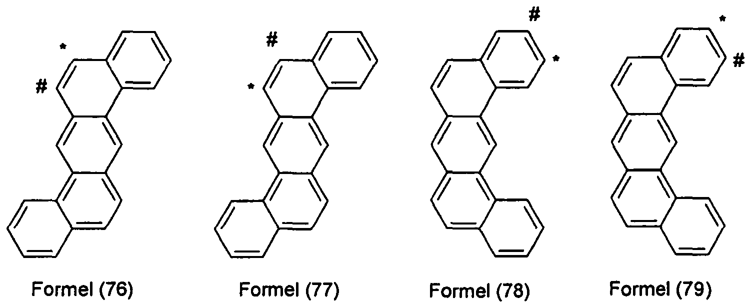

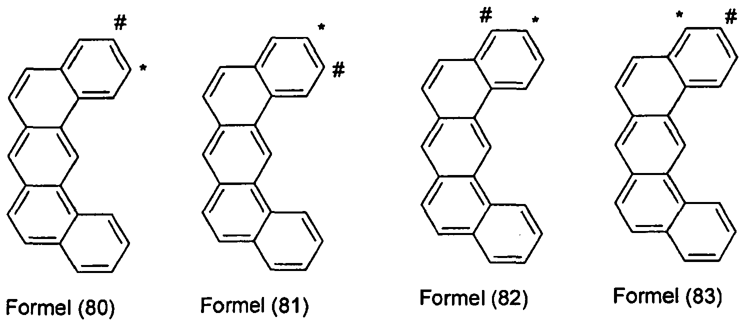

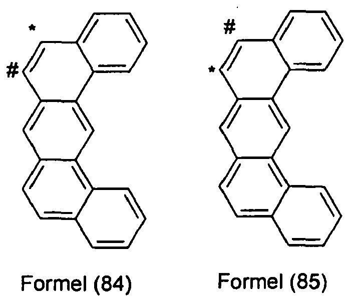

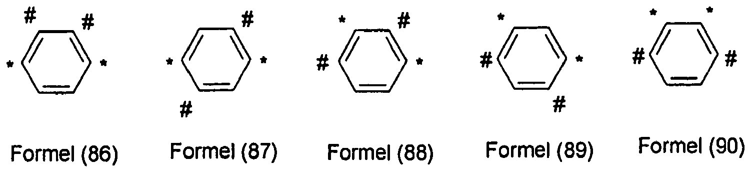

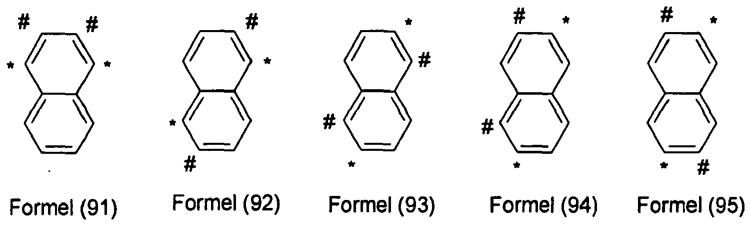

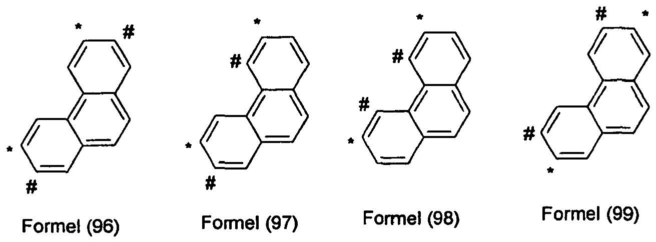

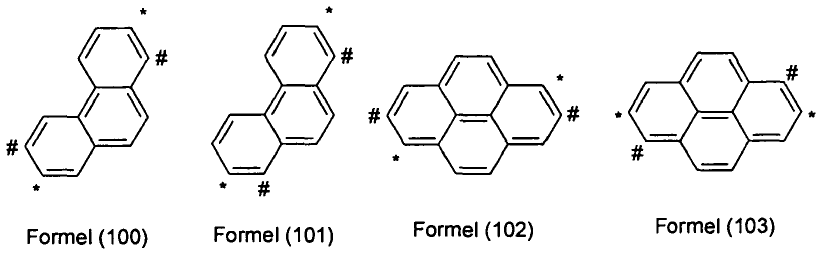

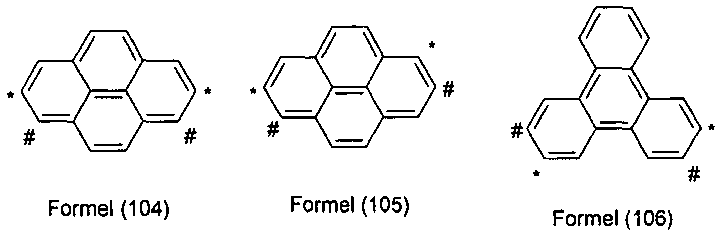

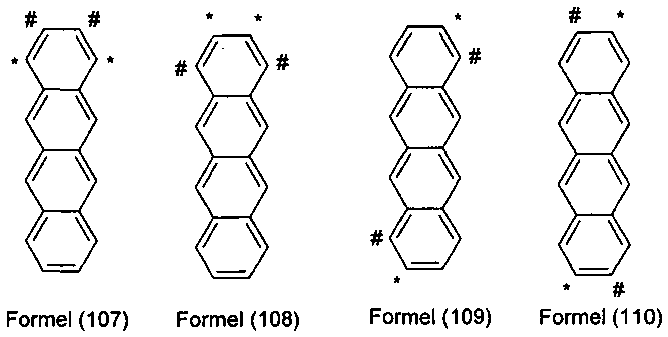

11. Organische Elektrolumineszenzvorrichtung nach einem oder mehreren der Ansprüche 1 bis 10, **dadurch gekennzeichnet, dass** das Hostmaterial für den blauen Dotanden ausgewählt ist aus der Gruppe bestehend aus Oligoarylenen, insbesondere Oligoarylenen enthaltend kondensierte aromatische Gruppen, Oligoarylenvinylenen, polypodalen Metallkomplexen, lochleitenden Verbindungen, elektronenleitenden Verbindungen, insbesondere Ketone, Phosphinoxide und Sulfoxide, Atropisomeren, Boronsäurederivaten oder Benzanthracenen, bevorzugt Oligoarylene, enthaltend Naphthalin, Anthracen, Benzanthracen, Benzphenanthren und/oder Pyren oder Atropisomere dieser Verbindungen, Ketonen, Phosphinoxiden und Sulfoxiden.

12. Organische Elektrolumineszenzvorrichtung nach Anspruch 11, **dadurch gekennzeichnet, dass** das Hostmaterial für den blauen Dotanden ausgewählt ist aus Verbindungen der Formel (142),
Ar⁴-(Ar⁵)ₚ-Ar⁶ Formel (142)
wobei Ar⁴, Ar⁵, Ar⁶ bei jedem Auftreten gleich oder verschieden eine Aryl- oder Heteroarylgruppe mit 5 bis 30 aromatischen Ringatomen ist, die mit einem oder mehreren Resten R¹ substituiert sein kann, und R¹ und p dieselbe Bedeutung haben, wie in Anspruch 8 beschrieben; dabei gilt, dass die Summe der π-Elektronen in Ar⁴, Ar⁵ und Ar⁶ mindestens 30 beträgt, wenn p = 1 ist, und mindestens 36 beträgt, wenn p = 2 ist, und mindestens 42 beträgt, wenn p = 3 ist.

13. Verfahren zur Herstellung einer organischen Elektrolumineszenzvorrichtung nach einem oder mehreren der Ansprüche 1 bis 12, **dadurch gekennzeichnet, dass** eine oder mehrere Schichten mit einem Sublimationsverfahren beschichtet werden oder dass eine oder mehrere Schichten mit dem OVPD (Organic Vapour Phase Deposition) Verfahren oder mit Hilfe einer Trägergassublimation beschichtet werden oder dass eine oder mehrere Schichten aus Lösung oder mit einem beliebigen Druckverfahren hergestellt werden.

## Claims

1. Organic electroluminescent device comprising, in this sequence, an anode, a first emitter layer, a second emitter layer which is a blue-emitting layer, where the blue-emitting layer comprises a host material in a proportion of 90 - 99.9% by vol. and a dopant in a proportion of 0.1 - 10% by vol., and a cathode, **characterised in that** the dopant has an HOMO, measured as indicated in Example 1 of the description, of less than -5.2 eV.

2. Organic electroluminescent device according to Claim 1, **characterised in that** the device has precisely two emitting layers, where the emitter layer on the anode side is a yellow- or orange-emitting emitter layer and where it is preferably a phosphorescent emitter layer.

3. Organic electroluminescent device according to Claim 1, **characterised in that** the device has at least three emitting layers, where one of these layers is a red- or orange-emitting emitter layer and one of the layers is a green-emitting emitter layer and the red- or orange-emitting layer is on the anode side and the green-emitting layer lies between the red-emitting layer and the blue-emitting layer, where the red- or orange-emitting layer and/or the green-emitting layer are preferably phosphorescent layers.

4. Organic electroluminescent device according to one or more of Claims 1 to 3, **characterised in that** the blue-emitting layer is a fluorescent layer.

5. Organic electroluminescent device according to one or more of Claims 1 to 4, **characterised in that** the blue dopant has an HOMO (highest occupied molecular orbital), measured as indicated in Example 1 of the description, of less than -5.3 eV, preferably less than -5.4 eV.

6. Organic electroluminescent device according to one or more of Claims 1 to 5, **characterised in that** the blue dopant has an LUMO (lowest unoccupied molecular orbital), measured as indicated in Example 1 of the description, of less than -2.3 eV, preferably less than -2.5 eV.

7. Organic electroluminescent device according to one or more of Claims 1 to 6, **characterised in that** the blue dopant is present in the blue-emitting layer in a concentration of 0.2 - 7% by vol., preferably 0.5 - 5% by vol., particularly preferably 0.8 - 3% by vol.

8. Organic electroluminescent device according to one or more of Claims 1 to 7, **characterised in that** the blue dopant is a compound of the following formula (1): 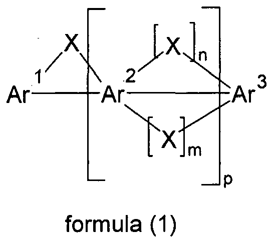 where the following applies to the symbols and indices used:
Ar¹, Ar², Ar³ are on each occurrence, identically or differently, an aryl or heteroaryl group having 5 to 30 aromatic ring atoms, which may be substituted by one or more radicals R¹;
X is on each occurrence, identically or differently, a group selected from BR², C(R²)₂, Si(R²)₂, C=O, C=NR², C=C(R²)₂, O, S, S=O, SO₂, NR², PR², P(=O)R² or P(=S)R²;
R¹, R² are on each occurrence, identically or differently, H, D, F, Cl, Br, I, C(=O)Ar⁴, P(=O)(Ar⁴)₂, S(=O)Ar⁴, S(=O)₂Ar⁴, CR²=CR²Ar⁴, CHO, CR³=C(R³)₂, CN, NO₂, Si(R³)₃, B(OR³)₂, B(R³)₂, B(N(R³)₂)₂, OSO₂R³, a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy or thioalkoxy group having 3 to 40 C atoms, each of which may be substituted by one or more radicals R³, where in each case one or more non-adjacent CH₂ groups may be replaced by R³C=CR³, C=C, Si(R³)₂, Ge(R³)₂, Sn(R³)₂, C=O, C=S, C=Se, C=NR³, P(=O)R³, SO, SO₂, NR³, O, S or CONR³ and where one or more H atoms may be replaced by F, Cl, Br, I, CN or NO₂, or an aromatic or heteroaromatic ring system having 5 to 60 aromatic ring atoms, which may in each case be substituted by one or more radicals R³, or a combination of these systems; two or more substituents R¹ or R² here may also form a mono- or polycyclic, aliphatic or aromatic ring system with one another;
R³ is on each occurrence, identically or differently, H, D or an aliphatic or aromatic hydrocarbon radical having 1 to 20 C atoms;
Ar⁴ is on each occurrence, identically or differently, an aromatic or heteroaromatic ring system having 5-30 aromatic ring atoms, which may be substituted by one or more non-aromatic radicals R¹; two radicals Ar on the same nitrogen or phosphorus atom may also be linked to one another here by a single bond or a bridge X;
m, n are 0 or 1, with the proviso that m + n = 1;
p is 1, 2, 3, 4, 5 or 6;
Ar¹, Ar² and X together form a five-membered ring or a six-membered ring, and Ar², Ar³ and X together form a five-membered ring or a six-membered ring;
the sum of all π electrons in the groups Ar¹, Ar² and Ar³ is preferably at least 28 if p = 1 and at least 34 if p = 2 and at least 40 if p = 3 and at least 46 if p = 4 and at least 52 if p = 5 and at least 58 if p = 6.

9. Organic electroluminescent device according to one or more of Claims 1 to 8, **characterised in that** the symbols Ar¹, Ar² and Ar³ stand, identically or differently on each occurrence, for an aryl or heteroaryl group having 5 to 22 aromatic ring atoms, in particular having 5 to 18 aromatic ring atoms, in particular selected from the group consisting of benzene, naphthalene, anthracene, phenan-threne, fluoranthene, naphthacene, benzanthracene, chrysene, pyrene, benzofluoranthene, triphenylene, perylene, dibenzanthra-cene, benzopyrene, picene, pentacene, pentaphene, benzophenanthrene, pyridine, pyrazine, pyrimidine, pyridazine, quinoline, isoquinoline, phenanthroline, acridine.

10. Organic electroluminescent device according to one or more of Claims 1 to 9, **characterised in that** the groups Ar¹ and Ar³ which form a five-membered ring with Ar² are the groups of the formulae (2) to (85) shown below, each of which may be substituted by one or more radicals R¹, and **in that** the groups Ar² are the groups of the formulae (86) to (110) shown below, each of which may be substituted by one or more radicals R¹; the symbol * stands for the position of the link from Ar¹ to Ar² or from Ar² to Ar³, and the symbol # stands for the position of the link to X:

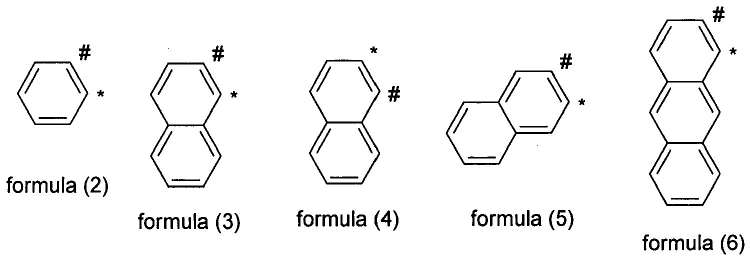

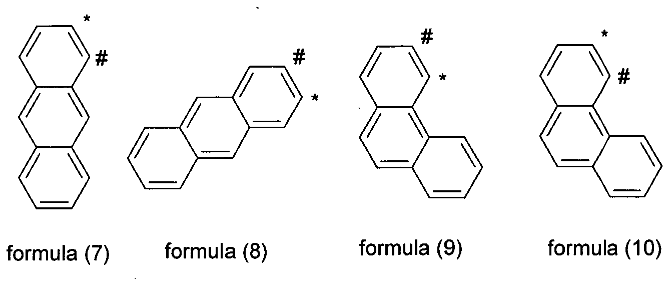

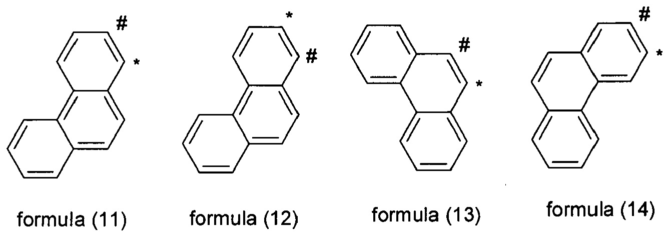

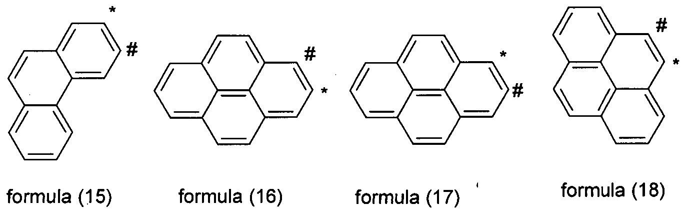

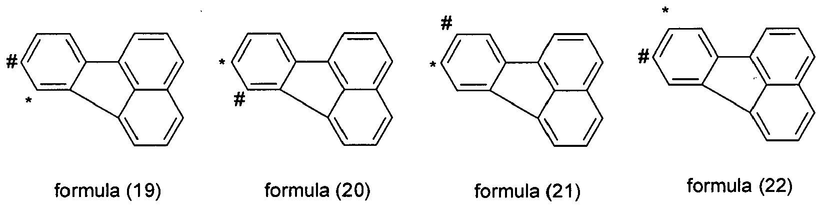

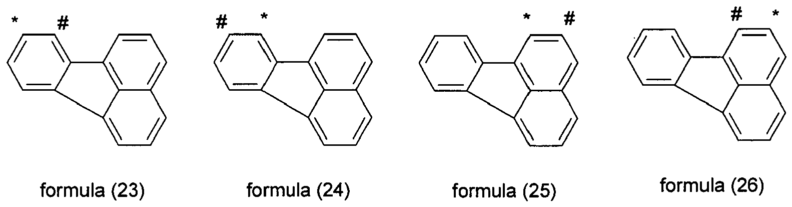

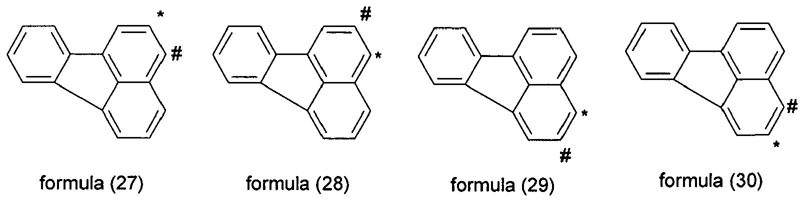

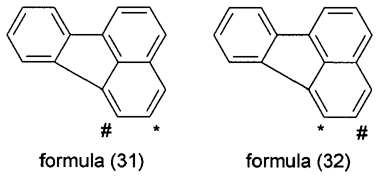

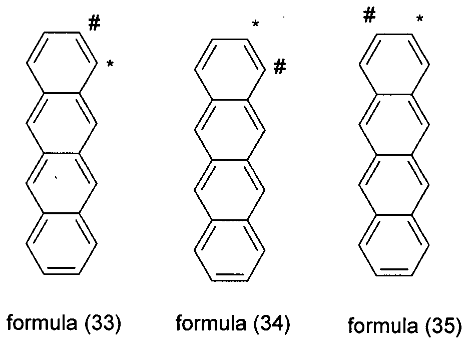

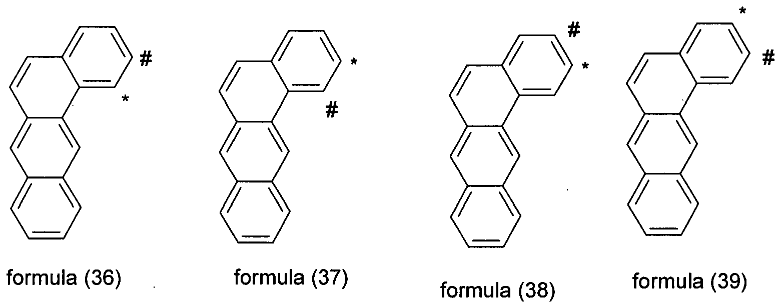

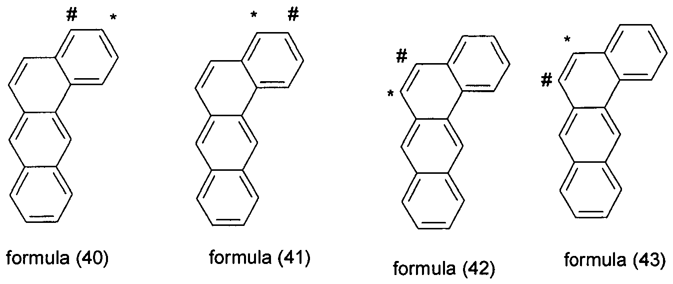

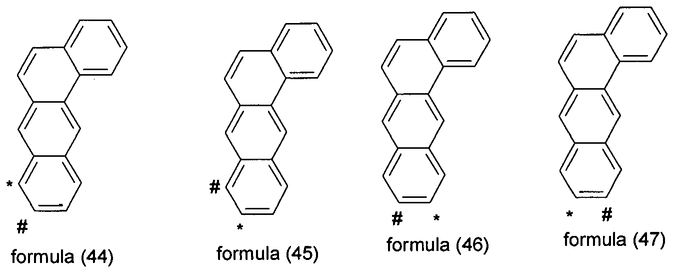

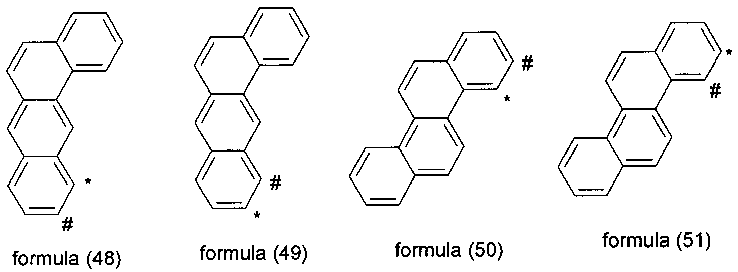

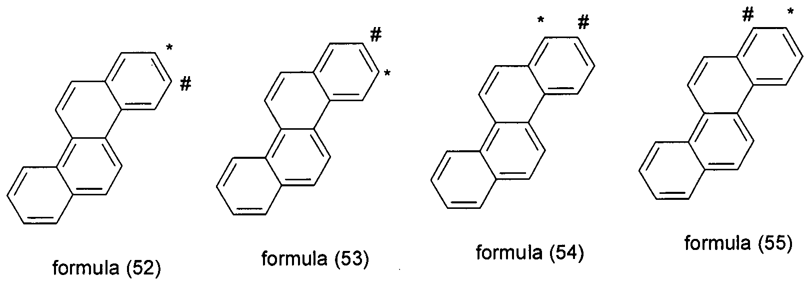

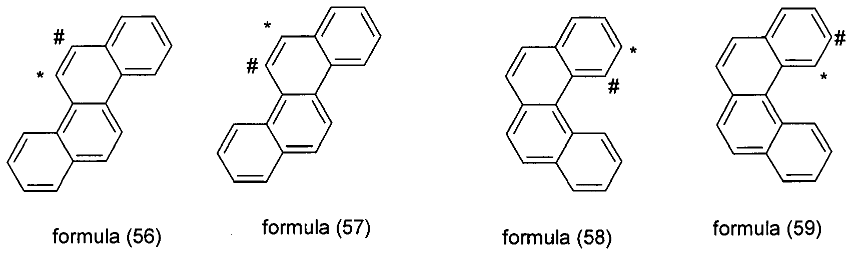

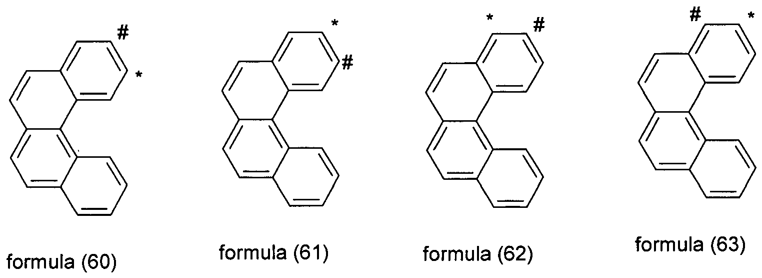

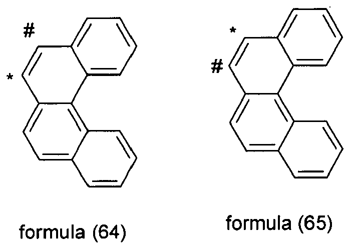

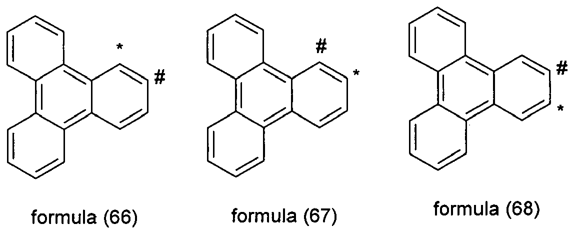

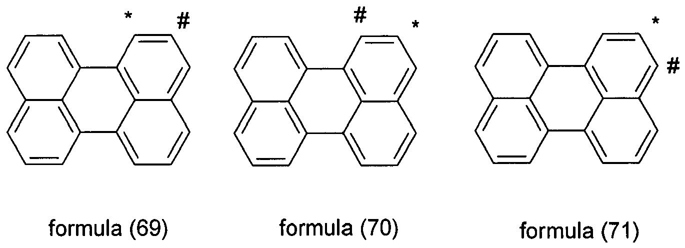

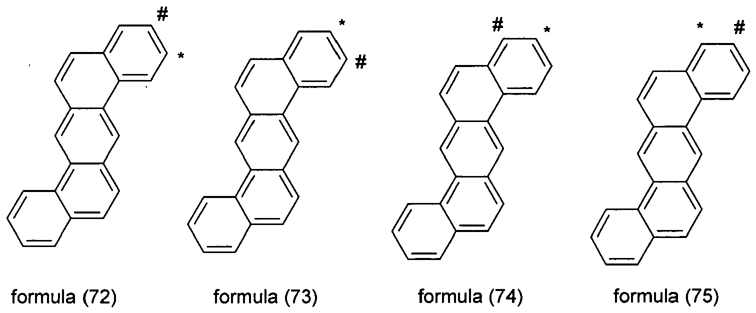

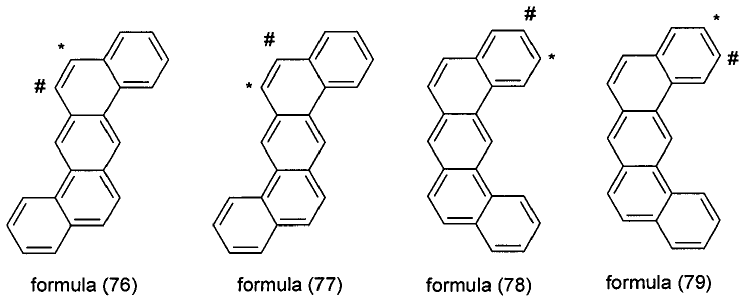

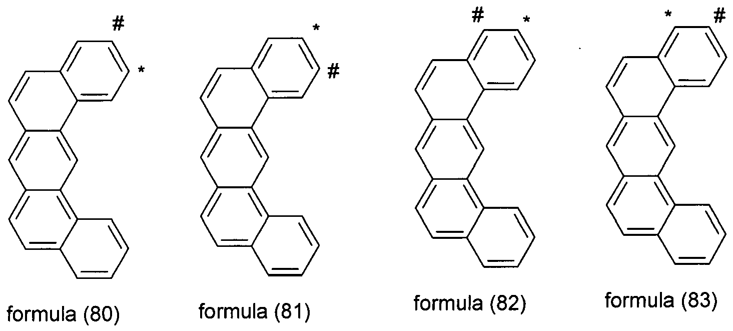

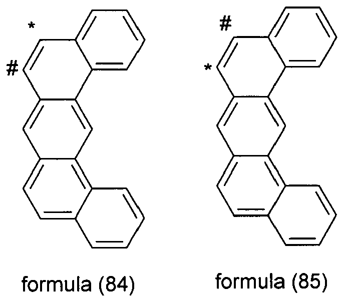

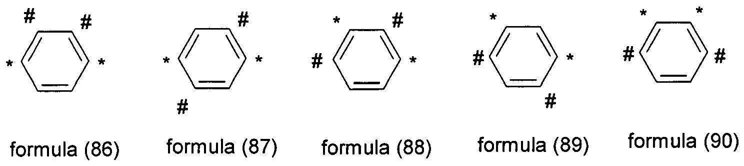

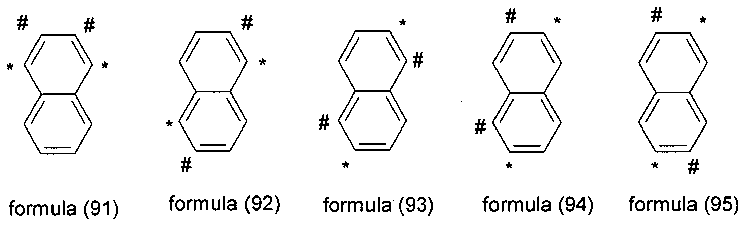

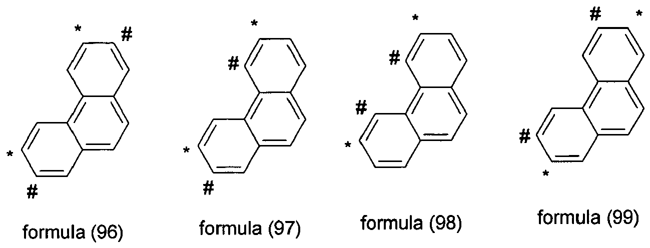

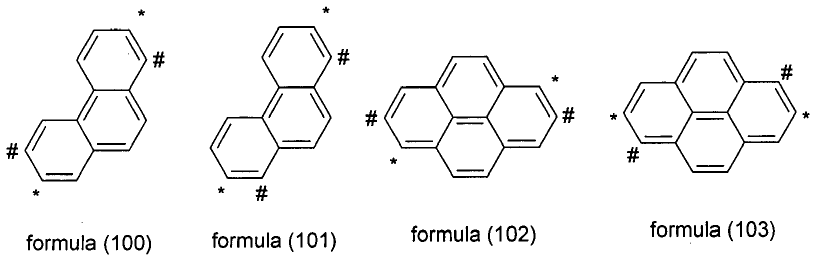

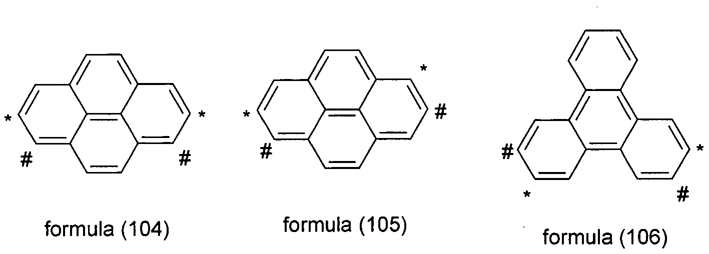

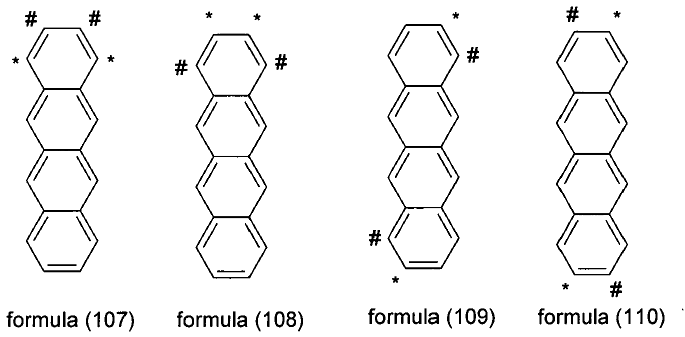

11. Organic electroluminescent device according to one or more of Claims 1 to 10, **characterised in that** the host material for the blue dopant is selected from the group consisting of oligoarylenes, in particular oligoarylenes containing condensed aromatic groups, oligo-arylenevinylenes, polypodal metal complexes, hole-conducting compounds, electron-conducting compounds, in particular ketones, phosphine oxides and sulfoxides, atropisomers, boronic acid derivatives or benzanthracenes, preferably oligoarylenes containing naphthalene, anthracene, benzanthracene, benzophenanthrene and/or pyrene, or atropisomers of these compounds, ketones, phosphine oxides and sulfoxides.

12. Organic electroluminescent device according to Claim 11, **characterised in that** the host material for the blue dopant is selected from compounds of the formula (142),
Ar⁴-(Ar⁵)ₚ-Ar⁶ formula (142)
where Ar⁴, Ar⁵, Ar⁶ are on each occurrence, identically or differently, an aryl or heteroaryl group having 5 to 30 aromatic ring atoms, which may be substituted by one or more radicals R¹, and R¹ and p have the same meaning as described in Claim 8; the sum of the π electrons in Ar⁴, Ar⁵ and Ar⁶ here is at least 30 if p = 1 and at least 36 if p = 2 and at least 42 if p = 3.

13. Process for the production of an organic electroluminescent device according to one or more of Claims 1 to 12, **characterised in that** one or more layers are applied by means of a sublimation process or **in that** one or more layers are applied by means of the OVPD (organic vapour phase deposition) process or with the aid of carrier-gas sublimation or **in that** one or more layers are produced from solution or by means of any desired printing process.

## Revendications

1. Dispositif électroluminescent organique comprenant, selon cette séquence, une anode, une première couche d'émetteur, une seconde couche d'émetteur qui est une couche d'émission de bleu, où la couche d'émission de bleu comprend un matériau hôte selon une proportion de 90 - 99,9% en volume et un dopant selon une proportion de 0,1 - 10% en volume, et une cathode, **caractérisé en ce que** le dopant présente une HOMO, mesurée comme indiqué selon l'exemple 1 de la description, inférieure à -5,2 eV.

2. Dispositif électroluminescent organique selon la revendication 1, **caractérisé en ce que** le dispositif comporte précisément deux couches d'émission, où la couche d'émetteur sur le côté d'anode est une couche d'émetteur d'émission de jaune ou d'orange et où il s'agit de façon préférable d'une couche d'émetteur phosphorescente.

3. Dispositif électroluminescent organique selon la revendication 1, **caractérisé en ce que** le dispositif comporte au moins trois couches d'émission, où l'une de ces couches est une couche d'émetteur d'émission de rouge ou d'orange et l'une des couches est une couche d'émetteur d'émission de vert et la couche d'émission de rouge ou d'orange est sur le côté d'anode et la couche d'émission de vert s'étend entre la couche d'émission de rouge et la couche d'émission de vert, où la couche d'émission de rouge ou d'orange et/ou la couche d'émission de vert est/sont de préférence des couches phosphorescentes.

4. Dispositif électroluminescent organique selon une ou plusieurs des revendications 1 à 3, **caractérisé en ce que** la couche d'émission de bleu est une couche fluorescente.

5. Dispositif électroluminescent organique selon une ou plusieurs des revendications 1 à 4, **caractérisé en ce que** le dopant de bleu présente une HOMO (orbite moléculaire occupée la plus haute), mesurée comme indiqué selon l'exemple 1 de la description, inférieure à -5,3 eV, de préférence inférieure à -5,4 eV.

6. Dispositif électroluminescent organique selon une ou plusieurs des revendications 1 à 5, **caractérisé en ce que** le dopant de bleu présente une LUMO (orbite moléculaire inoccupée la plus basse), mesurée comme indiqué selon l'exemple 1 de la description, inférieure à -2.3 eV, de préférence inférieure à -2,5 eV.

7. Dispositif électroluminescent organique selon une ou plusieurs des revendications 1 à 6, **caractérisé en ce que** le dopant de bleu est présent dans la couche d'émission de bleu selon une concentration de 0,2 - 7% en volume, de préférence de 0,5 - 5% en volume, de façon particulièrement préférable de 0,8 - 3% en volume.

8. Dispositif électroluminescent organique selon une ou plusieurs des revendications 1 à 7, **caractérisé en ce que** le dopant de bleu est un composé de la formule (1) qui suit : 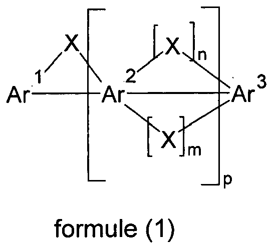 où ce qui suit s'applique aux symboles et indices utilisés :
Ar¹, Ar², Ar³ sont, pour chaque occurrence, de manière identique ou différente, un groupe aryle ou hétéroaryle comportant de 5 à 30 atomes de cycle aromatique, lequel peut être substitué par un ou plusieurs radicaux R¹;
X est, pour chaque occurrence, de manière identique ou différente, un groupe choisi parmi BR², C(R²)₂, Si(R²)₂, C=O, C=NR², C=C(R²)₂, O, S, S=O, SO₂, NR², PR², P(=O)R² ou P(=S)R²;
R¹, R² sont, pour chaque occurrence, de manière identique ou différente, H, D, F, Cl, Br, I, C(=O)Ar⁴, P(=O)(Ar⁴)₂, S(=O)Ar⁴, S(=O)₂Ar⁴, CR²=CR²Ar⁴, CHO, CR³=C(R³)₂, CN, NO₂, Si(R³)₃, B(OR³)₂, B(R³)₂, B(N(R³)₂)₂, OSO₂R³, un groupe alkyle, alcoxy ou thioalcoxy en chaîne droite comportant de 1 à 40 atomes de C ou un groupe alkényle ou alkynyle en chaîne droite comportant de 2 à 40 atomes de C ou un groupe alkyle, alkényle, alkynyle, alcoxy ou thioalcoxy ramifié ou cyclique comportant de 3 à 40 atomes de C, dont chacun peut être substitué par un ou plusieurs radicaux R³, où, dans chaque cas, un ou plusieurs groupes CH₂ non adjacents peut/peuvent être remplacé(s) par R³C=CR³, C=C, Si(R³)₂, Ge(R³)₂, Sn(R³)₂, C=O, C=S, C=Se, C=NR³, P(=O)R³, SO, SO₂, NR³, O, S ou CONR³ et où un ou plusieurs atomes de H peut/peuvent être remplacé(s) par F, Cl, Br, I, CN ou NO₂, ou un système de cycle aromatique ou hétéroaromatique comportant de 5 à 60 atomes de cycle aromatique, lequel peut dans chaque cas être substitué par un ou plusieurs radicaux R³, ou une combinaison de ces systèmes; deux substituants R¹ ou R² ou plus peuvent ici également former un système de cycle mono- ou polycyclique, aliphatique ou aromatique l'un avec l'autre ou les uns avec les autres ;
R³ est, pour chaque occurrence, de manière identique ou différente, H, D ou un radical hydrocarbone aliphatique ou aromatique comportant de 1 à 20 atomes de C ;
Ar⁴ est, pour chaque occurrence, de manière identique ou différente, un système de cycle aromatique ou hétéroaromatique comportant 5-30 atomes de cycle aromatique, lequel peut être substitué par un ou plusieurs radicaux non aromatiques R¹; deux radicaux Ar sur le même atome d'azote ou de phosphore peuvent également être liés l'un à l'autre ici par une liaison simple ou un pont X;
m, n sont 0 ou 1, étant entendu que m + n = 1 ;
p est 1, 2, 3, 4, 5 ou 6 ;
Ar¹, Ar² et X forment ensemble un cycle à cinq éléments ou un cycle à six éléments, et Ar², Ar³ et X forment ensemble un cycle à cinq éléments ou un cycle à six éléments ;
la somme de tous les électrons π dans les groupes Ar¹, Ar² et Ar³ est de préférence d'au moins 28 si p = 1 et d'au moins 34 si p = 2 et d'au moins 40 si p = 3 et d'au moins 46 si p = 4 et d'au moins 52 si p = 5 et d'au moins 58 si p = 6.

9. Dispositif électroluminescent organique selon une ou plusieurs des revendications 1 à 8, **caractérisé en ce que** les symboles Ar¹, Ar² et Ar³ représentent, de manière identique ou différente pour chaque occurrence, un groupe aryle ou hétéroaryle comportant de 5 à 22 atomes de cycle aromatique, en particulier comportant de 5 à 18 atomes de cycle aromatique, en particulier choisis parmi le groupe constitué par benzène, naphtalène, anthracène, phénanthrène, fluor-anthène, naphtacène, benzanthracène, chrysène, pyrène, benzo-fluoranthène, triphénylène, pérylène, dibenzanthracène, benzo-pyrène, picène, pentacène, pentaphène, benzophénanthrène, pyridine, pyrazine, pyrimidine, pyridazine, quinoline, isoquinoline, phén-anthroline, acridine.

10. Dispositif électroluminescent organique selon une ou plusieurs des revendications 1 à 9, **caractérisé en ce que** les groupes Ar¹ et Ar³ qui forment un cycle à cinq éléments avec Ar² sont les groupes des formules (2) à (85) présentées ci après, dont chacun peut être substitué par un ou plusieurs radicaux R¹, et **en ce que** les groupes Ar² sont les groupes des formules (86) à (110) présentées ci après, dont chacun peut être substitué par un ou plusieurs radicaux R¹; le symbole * représente la position de la liaison depuis Ar¹ jusqu'à Ar² ou depuis Ar² jusqu'à Ar³, et le symbole # représente la position de la liaison sur X:

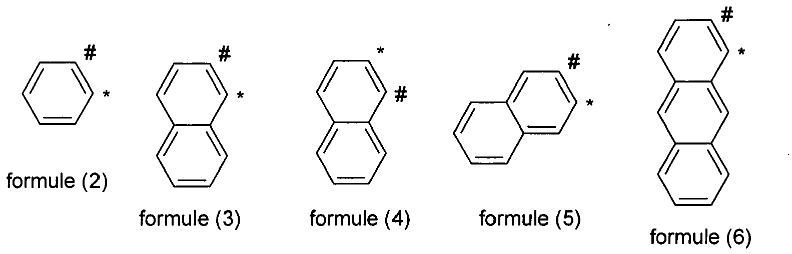

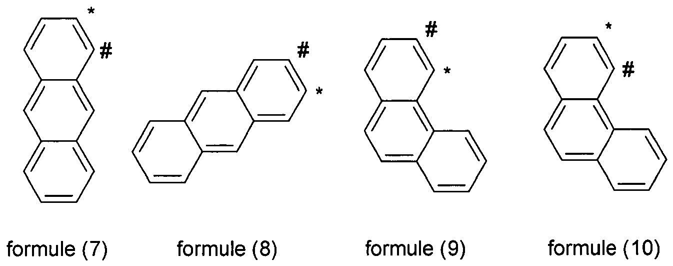

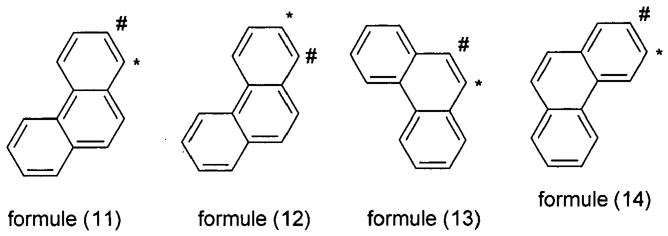

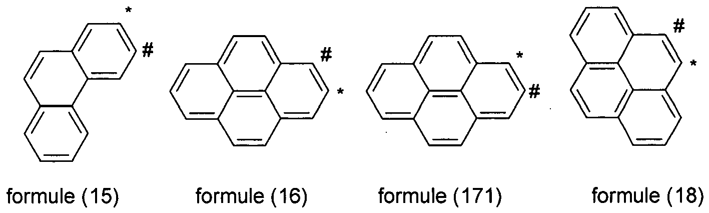

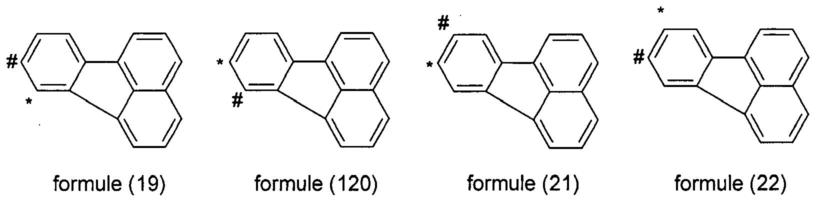

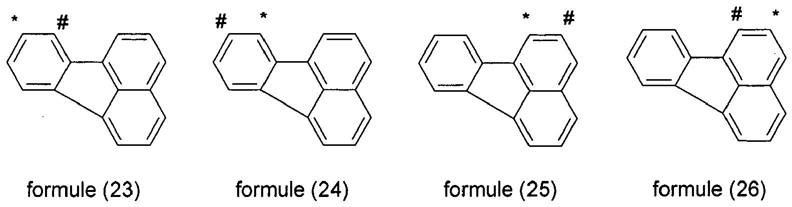

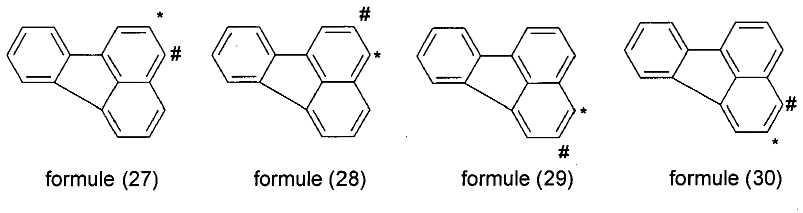

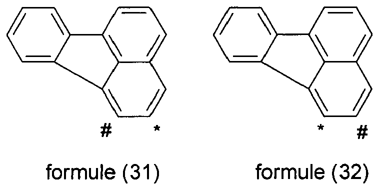

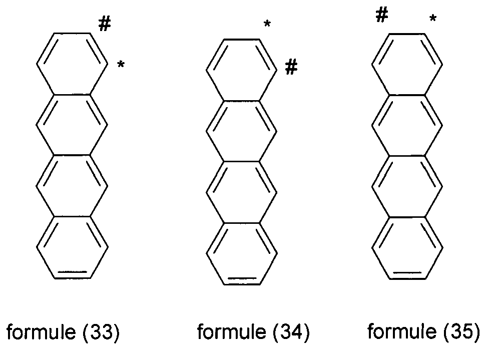

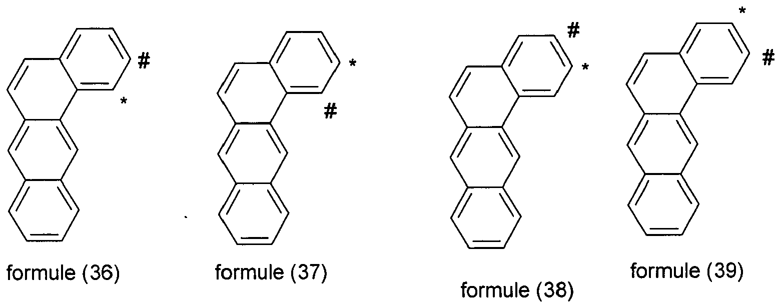

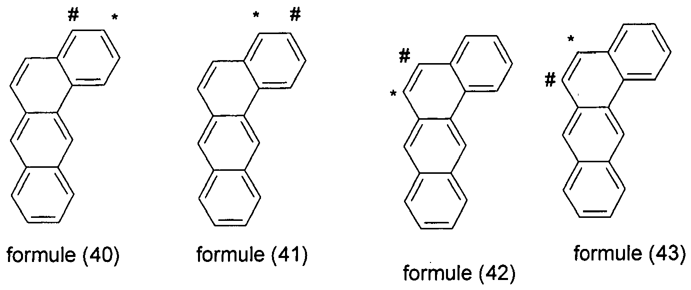

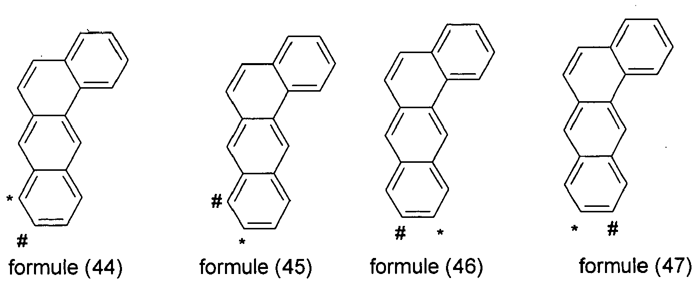

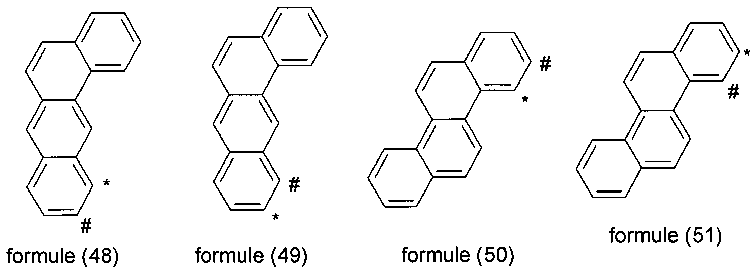

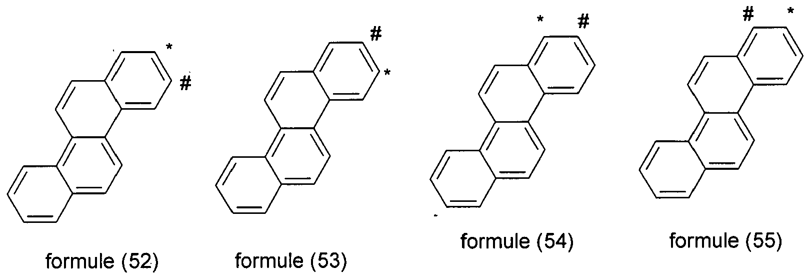

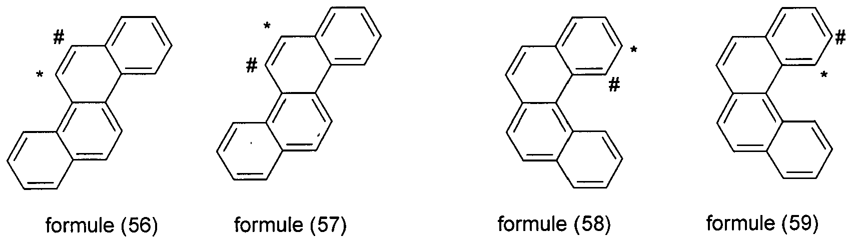

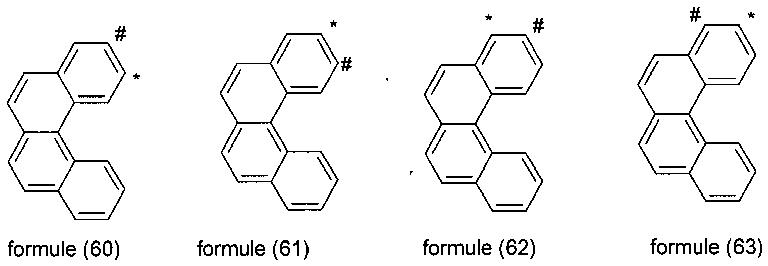

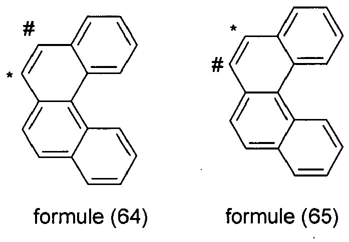

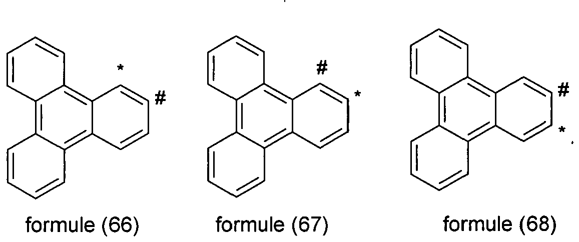

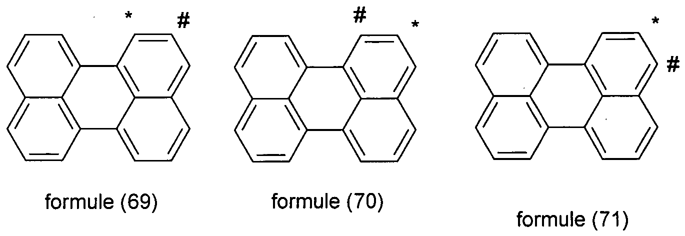

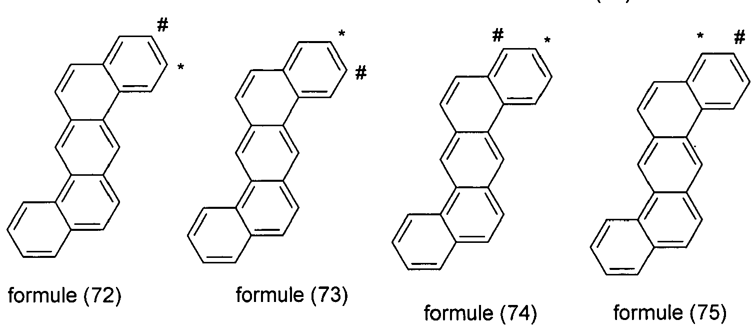

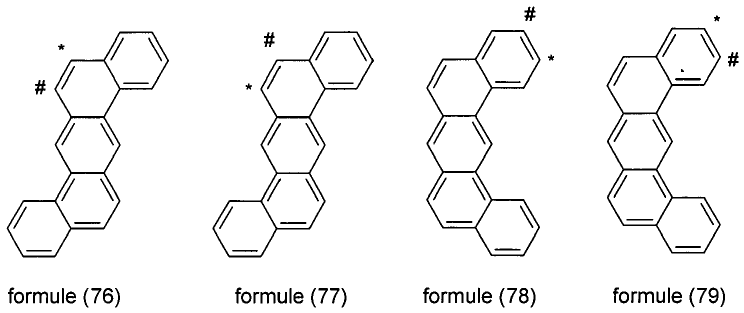

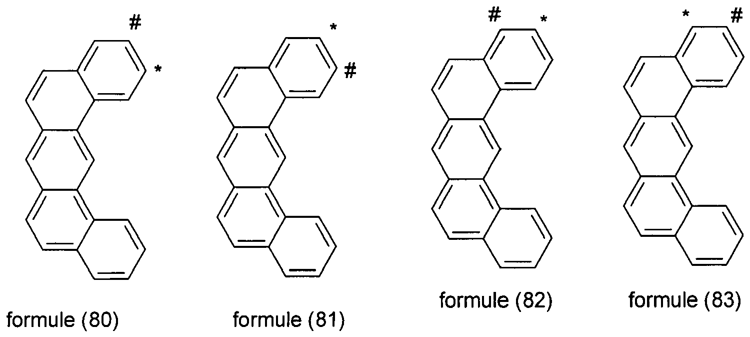

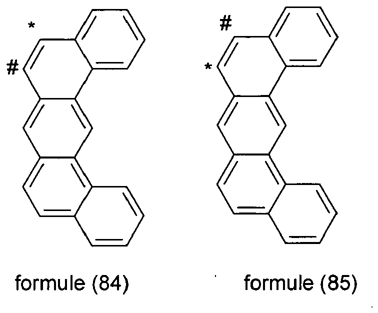

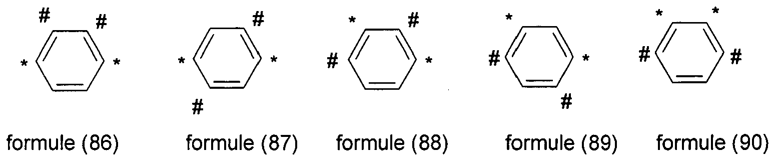

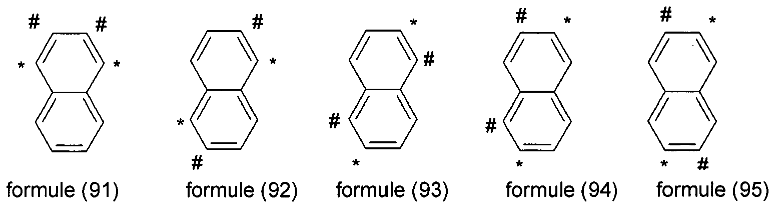

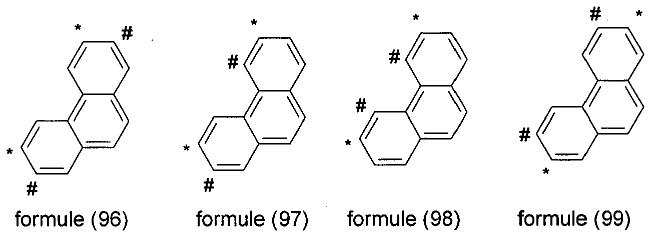

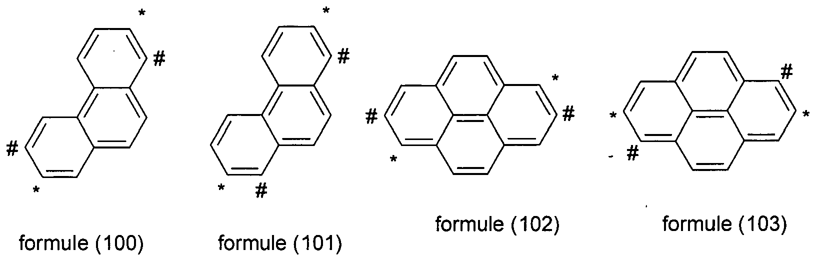

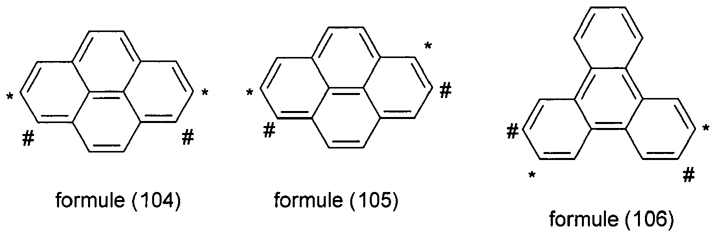

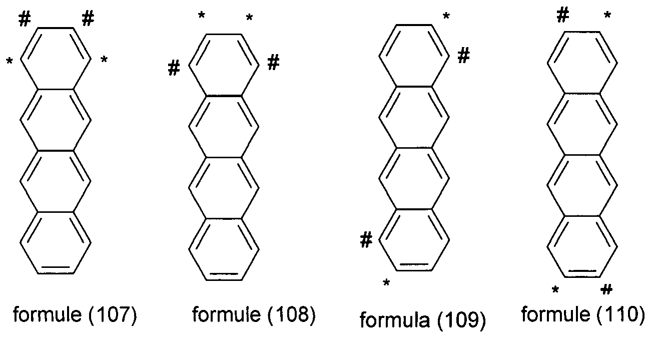

11. Dispositif électroluminescent organique selon une ou plusieurs des revendications 1 à 10, **caractérisé en ce que** le matériau hôte pour le dopant de bleu est choisi parmi le groupe constitué par des oligoarylènes, en particulier des oligoarylènes contenant des groupes aromatiques condensés, des oligoarylènevinylènes, des complexes métalliques polypodaux, des composés de conduction de trous, des composés de conduction d'électrons, en particulier des cétones, des oxydes et des sulfoxydes de phosphine, des atropisomères, des dérivés d'acide boronique ou des benzanthracènes, de préférence des oligoarylènes contenant naphtalène, anthracène, benzanthracène, benzophénanthrène et/ou pyrène, ou des atropisomères de ces composés, des cétones, des oxydes et des sulfoxydes de phosphine.

12. Dispositif électroluminescent organique selon la revendication 11, **caractérisé en ce que** le matériau hôte pour le dopant de bleu est choisi parmi les composés de la formule (142),
Ar⁴-(Ar⁵)ₚ-Ar⁶ formule (142)
dans laquelle Ar⁴, Ar⁵, Ar⁶ sont, pour chaque occurrence, de manière identique ou différente, un groupe aryle ou hétéroaryle comportant de 5 à 30 atomes de cycle aromatique, lequel peut être substitué par un ou plusieurs radicaux R¹, et R¹ et p présentent la même signification que décrit selon la revendication 8 ; la somme des électrons π dans Ar⁴, Ar⁵ et Ar⁶ est ici d'au moins 30 si p = 1 et d'au moins 36 si p = 2 et d'au moins 42 si p = 3.

13. Procédé pour la production d'un dispositif électroluminescent organique selon une ou plusieurs des revendications 1 à 12, **caractérisé en ce qu'**une ou plusieurs couches sont appliquées au moyen d'un procédé de sublimation ou **en ce qu'**une ou plusieurs couches sont appliquées au moyen du procédé OVPD (dépôt organique en phase vapeur) ou à l'aide d'une sublimation par gaz porteur ou **en ce qu'**une ou plusieurs couches sont produites à partir d'une solution ou au moyen de n'importe quel moyen d'impression souhaité.
